# EUROPEAN PATENT APPLICATION

(11) **EP 4 218 843 A2**
(43) Date of publication of application: **02.08.2023**
(21) Application number: 23155482.5
(22) Date of filing: 16.09.2020
(51) Int. Cl.: A61L 27/36, C07K 14/435, C12N 15/62

(54) **METHODS FOR ISOLATING SPIDER SILK PROTEINS VIA HIGH SHEAR SOLUBILIZATION**

(30) Priority: 16.09.2019 US 201962901053 P
(62) Divisional of application: 20865591.0
(71) Applicant: Bolt Threads, Inc., Emeryville, CA 94608 (US)
(72) Inventor: WHEATLEY, Robert, William, Emeryville, 94608 (US)
(74) Representative: Elkington and Fife LLP

(57) **Abstract**

The present disclosure relates to methods of isolating and purifying synthetic block copolymer proteins via the application of solvents and high shear microfluidization methods.

## Description

### BACKGROUND

Spider's silk polypeptides are large (>150kDa, >1000 amino acids) polypeptides that can be broken down into three domains: an N-terminal non-repetitive domain (NTD), the repeat domain (REP), and the C-terminal non-repetitive domain (CTD). The NTD and CTD are relatively small (-150, -100 amino acids respectively), well-studied, and are believed to confer to the polypeptide aqueous stability, pH sensitivity, and molecular alignment upon aggregation. NTD also has a strongly predicted secretion tag, which is often removed during heterologous expression. The repetitive region composes -90% of the natural polypeptide, and folds into the crystalline and amorphous regions that confer strength and flexibility to the silk fiber, respectively.

Silk polypeptides come from a variety of sources, including bees, moths, spiders, mites, and other arthropods. Some organisms make multiple silk fibers with unique sequences, structural elements, and mechanical properties. For example, orb weaving spiders have six unique types of glands that produce different silk polypeptide sequences that are polymerized into fibers tailored to fit an environmental or lifecycle niche. The fibers are named for the gland they originate from and the polypeptides are labeled with the gland abbreviation (e.g. "Ma") and "Sp" for spidroin (short for spider fibroin). In orb weavers, these types include Major Ampullate (MaSp, also called dragline), Minor Ampullate (MiSp), Flagelliform (Flag), Aciniform (AcSp), Tubuliform (TuSp), and Pyriform (PySp). This combination of polypeptide sequences across fiber types, domains, and variation amongst different genus and species of organisms leads to a vast array of potential properties that can be harnessed by commercial production of the recombinant fibers. To date, the vast majority of the work with recombinant silks has focused on the Major Ampullate Spidroins (MaSp).

Currently, recombinant silk fibers are not commercially available and, with a handful of exceptions, are not produced in microorganisms outside of *Escherichia coli* and other gram-negative prokaryotes. Recombinant silks produced to date have largely consisted either of polymerized short silk sequence motifs or fragments of native repeat domains, sometimes in combination with NTDs and/or CTDs.

However, in some instances recombinant silk polypeptides form undesirable insoluble aggregates during production and purification. Due to their ability to aggregate and form β-sheet structures, proteins based on silk sequences are difficult to solubilize. Solubilization of these proteins often requires harsh chemical conditions for biological molecules, such as high molarity chaotrope solutions. Methods to re-solubilize the peptides during purification often degrade the proteins, resulting in poor yield and fibers with low tenacity and poor hand feel. Improved methods to purify these polypeptides that result in increased solubility and recovery of the silk proteins are therefore required.

Provided herein are methods of solubilizing silk proteins via the application of high physical energy, such as the shear, impact, and cavitation generated by high energy fluid processor, in conditions where chemical solvation, including various chaotrope solutions, is insufficient.

### SUMMARY

In one aspect, provided herein are methods of isolating a recombinant spider silk protein from a host cell, comprising: providing an insoluble mass comprising a recombinant spider silk protein; adding the insoluble mass to an aqueous solution comprising a solvent; applying a shear force to the aqueous solution comprising the insoluble mass thereby solubilizing the recombinant spider silk protein in the aqueous solution.

In some embodiments, the shear force is applied via microfluidization.

In some embodiments, the microfluidization generates shear rates of about 6×10⁶ s⁻¹ to 10×10⁶ s⁻¹. In some embodiments, the microfluidization generates shear rates of at least about 6×10⁶ s⁻¹. In some embodiments, the microfluidization generates shear rates of at least about 10×10⁶ s⁻¹.

In some embodiments, the microfluidization is performed at between 20,000 psi to 30,000 psi. In some embodiments, the microfluidization is performed at 30,000 psi. In some embodiments, the microfluidization is performed at 23,000 psi.

In some embodiments, the microfluidizer an M-110P or an LM10 microfluidizer.

In some embodiments, the microfluidizer comprises a G10Z interaction chamber. In some embodiments, the microfluidizer comprises an F12Y interaction chamber.

In some embodiments, the shear force is applied at least twice. In some embodiments, the shear force is applied three times. In some embodiments, the shear force is the same in the at least two applications. In some embodiments, the shear force is different in the at least two applications.

In some embodiments, the insoluble mass is derived from a cell culture comprising a host cell, wherein the host cell expresses the recombinant spider silk protein.

In some embodiments, the method further comprises collecting the insoluble mass derived from the cell culture, wherein the insoluble mass comprises the recombinant spider silk protein.

In some embodiments, the solvent is a chaotropic agent. In some embodiments, the chaotropic agent is urea, guanidine thiocyanate (GdnSCN), or guanidine chloride (GdnHCL).

In some embodiments, the insoluble mass is added to the aqueous solution at about a 5%, 10%, 15%, 20%, 25%, or 30% insoluble mass to solvent volume.

In some embodiments, the chaotropic agent is present in the aqueous solution at a concentration from 0.1 - 10M. In some embodiments, the aqueous solution comprises about 10M urea, about 4M - 8M GdnHCl, or about 3M - 6M GdnSCN. In some embodiments, the aqueous solution comprises a chaotropic activity of no more than an aqueous solution comprising 10M urea, an aqueous solution comprising 8M GdnHCl, or an aqueous solution comprising 6M GdnSCN.

In some embodiments, the chaotropic activity is quantified using an agar-gelation assay.

In some embodiments, the aqueous solution comprises about a 15% insoluble portion mass to an 85% volume of 3M GdnSCN. In some embodiments, the aqueous solution comprises about a 15% insoluble portion mass to an 85% volume of 4M GdnHCl. In some embodiments, the aqueous solution comprises about a 15% insoluble portion mass to an 85% volume of 10M urea.

In some embodiments, the insoluble mass is incubated between 20° and 30°C. In some embodiments, the insoluble mass is incubated at room temperature. In some embodiments, the insoluble mass is incubated at no more than 30°C. In some embodiments, the insoluble portion is incubated in the aqueous solution comprising the solvent for 60 to 120 minutes.

In some embodiments, the insoluble mass comprises a cell pellet.

In some embodiments, collecting the insoluble mass derived from the cell pellet comprises lysing the host cell.

In some embodiments, lysing comprises heat treatment, chemical treatment, shear disruption, physical homogenization, sonication, or chemical homogenization.

In some embodiments, collecting the insoluble mass of the cell culture further comprises centrifuging the lysed cell to obtain a first cell pellet.

In some embodiments, collecting the insoluble mass further comprises: incubating the cell pellet with a solution comprising 4M urea at a 10: 1 urea volume to pellet mass ratio; and centrifuging the solution comprising 4M urea to obtain a second cell pellet prior to incubating the second cell pellet in the aqueous solution comprising a solvent.

In some embodiments, the method further comprises isolating the recombinant spider silk protein from the aqueous solution, thereby producing an isolated recombinant spider silk protein.

In some embodiments, the recombinant spider silk protein is a highly crystalline silk protein, a high beta sheet content silk protein, or a low solubility silk protein.

In some embodiments, the recombinant spider silk protein comprises the *Uloborus diversus* MiSP protein as set forth in SEQ ID NO: 23 .

In some embodiments, the recombinant spider silk protein has a solubility threshold of less than 90%, 80%, 70%, 60%, or 50% in a non-chaotropic solvent.

In some embodiments, the cell culture comprises a fungal, a bacterial, or a yeast cell. In some embodiments, the bacterial cell is *Escherichia coli.*

In some embodiments, an amount of isolated recombinant spider silk protein is measured using an ELISA. In some embodiments, an amount of isolated recombinant spider silk protein is measured using Size Exclusion Chromatography.

In some embodiments, the isolated recombinant spider silk protein is full-length recombinant spider silk protein.

In some embodiments, the isolated recombinant spider silk protein comprises at least 60%, at least 65%, at least 70%, at least 75%, at least 80%, at least 85%, at least 90%, or at least 95% full-length recombinant spider silk protein.

In some embodiments, an amount of full-length recombinant spider silk protein is measured using an ELISA. In some embodiments, an amount of full-length recombinant spider silk protein is measured using Size Exclusion Chromatography.

In some embodiments, the purity of the isolated recombinant spider silk protein is 5-10%, 10-15%, 15-20%, 20-25%, 25-30%, 30-35%, 35-40%, 45-50%, 50-55%, 55-60%, 60-65%, 65-70%, 70-75%, 75-80%, 80-85%, 85-90%, 09-95%, or 95-100%.

In another aspect, provided herein are methods of isolating a recombinant spider silk protein from a host cell, comprising: providing an insoluble mass comprising a recombinant spider silk protein; adding the insoluble mass to an aqueous solution comprising a solvent, wherein the aqueous solution comprises 15% (w/v) insoluble portion in a final 10M urea concentration; applying a shear force via microfluidization to the aqueous solution comprising the insoluble mass thereby solubilizing the recombinant spider silk protein in the aqueous solution; and isolating the recombinant spider silk protein from the aqueous solution, thereby producing an isolated recombinant spider silk protein.

In another aspect, provided herein are methods of isolating a recombinant spider silk protein from a host cell, comprising: providing an insoluble mass comprising a recombinant spider silk protein; adding the insoluble mass to an aqueous solution, wherein the aqueous solution comprises about a 15% (w/v) insoluble portion in a final 10M urea concentration; applying a shear force via microfluidization, wherein the shear force is about 10×10⁶ s⁻¹, to the aqueous solution thereby solubilizing the recombinant spider silk protein in the aqueous solution; and isolating the recombinant spider silk protein from the aqueous solution, thereby producing an isolated recombinant spider silk protein.

In another aspect, provided herein are compositions comprising a recombinant spider silk protein produced by the methods disclosed herein.

In some embodiments, the recombinant spider silk comprises at least 55%, at least 60%, at least 65%, at least 70%, at least 75%, at least 80%, at least 85%, at least 90%, at least 95%, or 100% full length recombinant spider silk.

In another aspect, provided herein are silk fibers comprising a recombinant spider silk protein produced by the methods disclosed herein.

### BRIEF DESCRIPTION OF THE SEVERAL VIEWS OF THE DRAWINGS

These and other features, aspects, and advantages of the present methods and compositions described herein will become better understood with regard to the following description, and accompanying drawings, where:
**FIG. 1A****,** shows the calculated shear rate as a function of pressure in various interaction chamber types. **FIG. 1B** shows the calculated flow rates as a function of pressure in various single slotted interaction chamber types.
**FIG. 2** shows the SEC plot of the *Uloborus diversus* silk protein MiSp (SEQ ID NO: 23) after extraction with 10M urea and microfluidization processing. Arrow points at the MiSp protein peak.

### DETAILED DESCRIPTION

### Definitions

Terms used in the claims and specification are defined as set forth below unless otherwise specified.

Unless otherwise defined herein, scientific and technical terms used in connection with the present methods and compositions described herein shall have the meanings that are commonly understood by those of ordinary skill in the art. Further, unless otherwise required by context, singular terms shall include the plural and plural terms shall include the singular. Generally, nomenclatures used in connection with, and techniques of, biochemistry, enzymology, molecular and cellular biology, microbiology, genetics and polypeptide and nucleic acid chemistry and hybridization described herein are those well-known and commonly used in the art.

The methods and techniques described herein are generally performed according to conventional methods well known in the art and as described in various general and more specific references that are cited and discussed throughout the present specification unless otherwise indicated. *See, e.g.,* Sambrook et al., Molecular Cloning: A Laboratory Manual, 2d ed., Cold Spring Harbor Laboratory Press, Cold Spring Harbor, N.Y. (1989); Ausubel et al., Current Protocols in Molecular Biology, Greene Publishing Associates (1992, and Supplements to 2002); Harlow and Lane, Antibodies: A Laboratory Manual, Cold Spring Harbor Laboratory Press, Cold Spring Harbor, N.Y. (1990); Taylor and Drickamer, Introduction to Glycobiology, Oxford Univ. Press (2003); Worthington Enzyme Manual, Worthington Biochemical Corp., Freehold, N.J.; Handbook of Biochemistry: Section A Proteins, Vol I, CRC Press (1976); Handbook of Biochemistry: Section A Proteins, Vol II, CRC Press (1976); Essentials of Glycobiology, Cold Spring Harbor Laboratory Press (1999).

All publications, patents and other references mentioned herein are hereby incorporated by reference in their entireties.

The following terms, unless otherwise indicated, shall be understood to have the following meanings:

The term **"in vitro"** refers to processes that occur in a living cell growing separate from a living organism, e.g., growing in tissue culture.

The term **"in vivo"** refers to processes that occur in a living organism.

The term **"clarifying"** as use herein refers to a method removing host cell biomass, such as whole cells, lysed cells, membranes, lipids, organelles, nuclei, non-spider silk proteins, or any other undesirable cell part or product, or any other undesirable portion of a cell culture. Clarifying may also refer to removing impurities from a partially purified or isolated spider silk composition. Impurities may include, but are not limited to, non-spider silk proteins, degraded spider silk proteins, large aggregates of proteins, chemicals used during the purification and isolation process, or any other undesirable material.

The term **"purity"** as used herein refers to the amount of substantially full-length isolated recombinant spider silk protein as a portion of all isolated components, such as partial or degraded isolated recombinant spider silk proteins, lipids, proteins, membranes, or other molecules in a sample, such as an extracted sample. A full length recombinant protein is at least 90-100% the length of the known full length protein. In some embodiments, a full length recombinant spider silk protein is at least 90%, 91%, 92%, 93%, 94%, 95%, 96%, 97%, 98%, 99%, or 100% the length of the known full length protein.

The term **"yield"** as used herein refers to the amount of total spider silk recovered, including fragments of spider silk protein and substantially full length spider silk, as compared to the starting amount of spider silk.

The term **"shear force"** refers to a force that acts in a direction parallel or tangential to a surface or to a planar cross section of a body or mass. The term **"shear rate"** for fluids refers to the rate of change of velocity at which one layer of fluid passes over an adjacent layer. For example, a shear rate occurs when two parallel planes of fluid move at different velocities.

The term **"soluble silk protein"** refers to the protein that remains in the supernatant after rigorous centrifugation. An example of rigorous centrifugation is a 50 mL aliquot sample of silk protein in a 50 mL conical centrifuge tube centrifuged at 15,000 × g for 20 min at room temperature.

The term **"polynucleotide"** or **"nucleic acid molecule"** refers to a polymeric form of nucleotides of at least 10 bases in length. The term includes DNA molecules (e.g., cDNA or genomic or synthetic DNA) and RNA molecules (e.g., mRNA or synthetic RNA), as well as analogs of DNA or RNA containing non-natural nucleotide analogs, non-native internucleoside bonds, or both. The nucleic acid can be in any topological conformation. For instance, the nucleic acid can be single-stranded, double-stranded, triple-stranded, quadruplexed, partially double-stranded, branched, hairpinned, circular, or in a padlocked conformation.

Unless otherwise indicated, and as an example for all sequences described herein under the general format "SEQ ID NO:", "nucleic acid comprising SEQ ID NO: 1" refers to a nucleic acid, at least a portion of which has either (i) the sequence of SEQ ID NO: 1, or (ii) a sequence complementary to SEQ ID NO: 1. The choice between the two is dictated by the context. For instance, if the nucleic acid is used as a probe, the choice between the two is dictated by the requirement that the probe be complementary to the desired target.

An **"isolated"** RNA, DNA or a mixed polymer is one which is substantially separated from other cellular components that naturally accompany the native polynucleotide in its natural host cell, *e.g.,* ribosomes, polymerases and genomic sequences with which it is naturally associated.

The term **"recombinant"** refers to a biomolecule, *e.g.,* a gene or polypeptide, that (1) has been removed from its naturally occurring environment, (2) is not associated with all or a portion of a polynucleotide in which the gene is found in nature, (3) is operatively linked to a polynucleotide which it is not linked to in nature, or (4) does not occur in nature. The term "recombinant" can be used in reference to cloned DNA isolates, chemically synthesized polynucleotide analogs, or polynucleotide analogs that are biologically synthesized by heterologous systems, as well as polypeptides and/or mRNAs encoded by such nucleic acids.

As used herein, an endogenous nucleic acid sequence in the genome of an organism (or the encoded polypeptide product of that sequence) is deemed **"recombinant"** herein if a heterologous sequence is placed adjacent to the endogenous nucleic acid sequence, such that the expression of this endogenous nucleic acid sequence is altered. In this context, a heterologous sequence is a sequence that is not naturally adjacent to the endogenous nucleic acid sequence, whether or not the heterologous sequence is itself endogenous (originating from the same host cell or progeny thereof) or exogenous (originating from a different host cell or progeny thereof). By way of example, a promoter sequence can be substituted (*e.g.,* by homologous recombination) for the native promoter of a gene in the genome of a host cell, such that this gene has an altered expression pattern. This gene would now become "recombinant" because it is separated from at least some of the sequences that naturally flank it. In an embodiment, a heterologous nucleic acid molecule is not endogenous to the organism. In further embodiments, a heterologous nucleic acid molecule is a plasmid or molecule integrated into a host chromosome by homologous or random integration.

A nucleic acid is also considered **"recombinant"** if it contains any modifications that do not naturally occur to the corresponding nucleic acid in a genome. For instance, an endogenous coding sequence is considered "recombinant" if it contains an insertion, deletion or a point mutation introduced artificially, *e.g.,* by human intervention. A "recombinant nucleic acid" also includes a nucleic acid integrated into a host cell chromosome at a heterologous site and a nucleic acid construct present as an episome.

The term **"percent sequence identity"** in the context of nucleic acid sequences refers to the quantitative value of an alignment of the residues in the two sequences when aligned for maximum correspondence. The length of sequence identity comparison may be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides. There are a number of different algorithms known in the art which can be used to measure nucleotide sequence identity. For instance, polynucleotide sequences can be compared using FASTA, Gap or Bestfit, which are programs in Wisconsin Package Version 10.0, Genetics Computer Group (GCG), Madison, Wis. FASTA provides alignments and percent sequence identity of the regions of the best overlap between the query and search sequences. Pearson, Methods Enzymol. 183:63-98 (1990) (hereby incorporated by reference in its entirety). For instance, percent sequence identity between nucleic acid sequences can be determined using FASTA with its default parameters (a word size of 6 and the NOPAM factor for the scoring matrix) or using Gap with its default parameters as provided in GCG Version 6.1, herein incorporated by reference. Alternatively, sequences can be compared using the computer program, BLAST (Altschul et al., J. Mol. Biol. 215:403-410 (1990); Gish and States, Nature Genet. 3:266-272 (1993); Madden et al., Meth. Enzymol. 266:131-141 (1996); Altschul et al., Nucleic Acids Res. 25:3389-3402 (1997); Zhang and Madden, Genome Res. 7:649-656 (1997)), especially blastp or tblastn (Altschul et al., Nucleic Acids Res. 25:3389-3402 (1997)).

The term **"substantial homology"** or **"substantial similarity,"** when referring to a nucleic acid or fragment thereof, indicates that, when optimally aligned with appropriate nucleotide insertions or deletions with another nucleic acid (or its complementary strand), there is nucleotide sequence identity in at least about 76%, 80%, 85%, preferably at least about 90%, and more preferably at least about 95%, 96%, 97%, 98% or 99% of the nucleotide bases, as measured by any well-known algorithm of sequence identity, such as FASTA, BLAST or Gap, as discussed above.

Nucleic acids (also referred to as polynucleotides) can include both sense and antisense strands of RNA, cDNA, genomic DNA, and synthetic forms and mixed polymers of the above. They can be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, *etc*.), pendent moieties (e.g., polypeptides), intercalators (e.g., acridine, psoralen, *etc*.), chelators, alkylators, and modified linkages (e.g., alpha anomeric nucleic acids, *etc*.) Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule. Other modifications can include, for example, analogs in which the ribose ring contains a bridging moiety or other structure such as the modifications found in "locked" nucleic acids.

The term **"mutated"** when applied to nucleic acid sequences means that nucleotides in a nucleic acid sequence may be inserted, deleted or changed compared to a reference nucleic acid sequence. A single alteration may be made at a locus (a point mutation) or multiple nucleotides may be inserted, deleted or changed at a single locus. In addition, one or more alterations may be made at any number of loci within a nucleic acid sequence. A nucleic acid sequence may be mutated by any method known in the art including but not limited to mutagenesis techniques such as "error-prone PCR" (a process for performing PCR under conditions where the copying fidelity of the DNA polymerase is low, such that a high rate of point mutations is obtained along the entire length of the PCR product; see, *e.g.,* Leung et al., Technique, 1:11-15 (1989) and Caldwell and Joyce, PCR Methods Applic. 2:28-33 (1992)); and "oligonucleotide-directed mutagenesis" (a process which enables the generation of site-specific mutations in any cloned DNA segment of interest; see, e.g., Reidhaar-Olson and Sauer, Science 241:53-57 (1988)).

The term **"vector"** as used herein is intended to refer to a nucleic acid molecule capable of transporting another nucleic acid to which it has been linked. One type of vector is a **"plasmid,"** which generally refers to a circular double stranded DNA loop into which additional DNA segments may be ligated, but also includes linear double-stranded molecules such as those resulting from amplification by the polymerase chain reaction (PCR) or from treatment of a circular plasmid with a restriction enzyme. Other vectors include cosmids, bacterial artificial chromosomes (BAC) and yeast artificial chromosomes (YAC). Another type of vector is a viral vector, wherein additional DNA segments may be ligated into the viral genome (discussed in more detail below). Certain vectors are capable of autonomous replication in a host cell into which they are introduced (e.g., vectors having an origin of replication which functions in the host cell). Other vectors can be integrated into the genome of a host cell upon introduction into the host cell, and are thereby replicated along with the host genome. Moreover, certain preferred vectors are capable of directing the expression of genes to which they are operatively linked. Such vectors are referred to herein as **"recombinant expression vectors"** (or simply **"expression vectors").**

The term **"expression system"** as used herein includes vehicles or vectors for the expression of a gene in a host cell as well as vehicles or vectors which bring about stable integration of a gene into the host chromosome.

**"Operatively linked"** or **"operably linked"** expression control sequences refers to a linkage in which the expression control sequence is contiguous with the gene of interest to control the gene of interest, as well as expression control sequences that act in trans or at a distance to control the gene of interest.

The term **"expression control sequence"** as used herein refers to polynucleotide sequences which are necessary to affect the expression of coding sequences to which they are operatively linked. Expression control sequences are sequences which control the transcription, post-transcriptional events and translation of nucleic acid sequences. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (*e.g.,* ribosome binding sites); sequences that enhance polypeptide stability; and when desired, sequences that enhance polypeptide secretion. The nature of such control sequences differs depending upon the host organism; in prokaryotes, such control sequences generally include promoter, ribosomal binding site, and transcription termination sequence. The term **"control sequences"** is intended to include, at a minimum, all components whose presence is essential for expression, and can also include additional components whose presence is advantageous, for example, leader sequences and fusion partner sequences.

The term **"promoter,"** as used herein, refers to a DNA region to which RNA polymerase binds to initiate gene transcription, and positions at the 5' direction of an mRNA transcription initiation site.

The term **"recombinant host cell"** (or simply "host cell"), as used herein, is intended to refer to a cell into which a recombinant vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A recombinant host cell may be an isolated cell or cell line grown in culture or may be a cell which resides in a living tissue or organism.

The term **"polypeptide"** encompasses both naturally-occurring and non-naturally-occurring proteins, and fragments, mutants, derivatives and analogs thereof. A polypeptide may be monomeric or polymeric. Further, a polypeptide may comprise a number of different domains each of which has one or more distinct activities.

As used herein, the term **"molecule"** means any compound, including, but not limited to, a small molecule, peptide, polypeptide, sugar, nucleotide, nucleic acid, polynucleotide, lipid, *etc.,* and such a compound can be natural or synthetic.

The term **"block"** or **"repeat unit"** as used herein refers to a subsequence greater than approximately 12 amino acids of a natural silk polypeptide that is found, possibly with modest variations, repeatedly in the natural silk polypeptide sequence and serves as a basic repeating unit in the silk polypeptide sequence. Blocks may, but do not necessarily, include very short "**motifs**." A "**motif**" as used herein refers to an approximately 2-10 amino acid sequence that appears in multiple blocks. For example, a motif may consist of the amino acid sequence GGA, GPG, or AAAAA (SEQ ID NO: 38). A sequence of a plurality of blocks is a **"block co-polymer."**

As used herein, the term **"repeat domain"** refers to a sequence selected from the set of contiguous (unbroken by a substantial non-repetitive domain, excluding known silk spacer elements) repetitive segments in a silk polypeptide. Native silk sequences generally contain one repeat domain. In some embodiments, there is one repeat domain per silk molecule. A **"macro-repeat"** as used herein is a naturally occurring repetitive amino acid sequence comprising more than one block. In an embodiment, a macro-repeat is repeated at least twice in a repeat domain. In a further embodiment, the two repetitions are imperfect. A "quasi-repeat" as used herein is an amino acid sequence comprising more than one block, such that the blocks are similar but not identical in amino acid sequence.

A **"repeat sequence"** or "**R**" as used herein refers to a repetitive amino acid sequence. In an embodiment, a repeat sequence includes a macro-repeat or a fragment of a macro-repeat. In another embodiment, a repeat sequence includes a block. In a further embodiment, a single block is split across two repeat sequences.

The term **"about"** indicates and encompasses an indicated value and a range above and below that value. In certain embodiments, the term "about" indicates the designated value ± 10%, ± 5%, or ± 1%. In certain embodiments, where applicable, the term "about" indicates the designated value(s) ± one standard deviation of that value(s).

It must be noted that, as used in the specification and the appended claims, the singular forms "a," "an" and "the" include plural referents unless the context clearly dictates otherwise.

Ranges recited herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50. In addition, a range of 2-5% includes 2% and 5%, and any number or fraction of a number in between, for example: 2.25%, 2.5%, 2.75%, 3%, 3.25%, 3.5%, 3.75%, 4%, 4.25%, 4.5%, and 4.75%.

### Methods for Solubilizing and Purifying a Recombinant Protein

Recombinant spider silk protein expressed in cell culture must be purified away from the cell components. In some instances, the silk protein is trapped in insoluble cell debris, or forms insoluble silk protein aggregates. Insoluble silk protein is difficult to purify and results in decreased recombinant silk protein recovery. In addition, some isolated spider silk protein solids can also be insoluble. For example, MBI 18B silk powder has low solubility, and some model spider silks, such as *U. diversus* MiSp is known to be highly insoluble. In such cases, various methods can be applied to the insoluble mass, aggregate, or silk solid to release the silk protein and solubilize it for purification, resulting in increased recovery of the recombinant silk protein. In addition, even in conditions where silk is soluble (*i.e.,* where the silk protein dissolution is thermodynamically favored) the rate of solubilization may be slow. This slow rate may be due to a high activation energy required for the solubilization reaction, or that the mass transport of the silk out of the cell debris particles is rate limiting. In both cases, where the silk aggregates are insoluble, or where the silk is soluble but slow to solubilize, the application of a high physical energy process such as a high shear force via homogenization or microfluidization can promote solubilization of the silk protein. High shear forces add energy to the solution to increase the reaction rate, thereby overcoming the high activation energy, or by breaking up the cell particles and silk protein aggregates to reduce the mass transport effects. Thus, the application of physical energy in the form of shear forces can increasing the solubility, and therefore the recovery, of recombinant spider silk protein.

Described herein are methods for solubilizing, isolating, and purifying a recombinant spider silk protein via application of a high physical energy process such as shear force, shear rate, impact, and cavitation. In some embodiments, the high physical energy process is applied via homogenization or microfluidization. The application of the high physical energy process to a recombinant protein results in increased solubilization of the protein in a an aqueous solution, thereby allowing for increased purification and recovery of the recombinant protein.

In some embodiments, the insoluble mass is a cell pellet. In some embodiments, the insoluble mass is a cell lysate. In some embodiments, the insoluble mass is an isolated spider silk solid, mass powder, or extrudate. A silk solid or recombinant silk solid is an isolated recombinant spider silk composition, such as a fiber, extrudate, powder, or pellet. An extrudate is an extruded recombinant spider silk composition that has been extruded through a spinneret.

### Physical Energy and Shear Force

Physical energy can be applied to the insoluble recombinant spider silk protein to increase the solubility of the protein. Physical energy is kinetic or mechanical energy that is transferred to the insoluble recombinant spider silk, via the application of mechanical forces, such as compression or constriction, pressure, fluid flow, impact, cavitation, shear force, shear rate, shear stress, stretching, or any combination thereof, or any other appropriate mechanical force known in the art. This application of mechanical energy increases the solubility of the recombinant proteins in solution. Generally, any method that applies kinetic or mechanical energy that induces controlled injury to the recombinant protein may be used to solubilize the recombinant protein. The increased solubility of the insoluble recombinant protein can be caused by, for example, pressure induced by mechanical strain or shear forces, subjecting the recombinant protein or cell lysate to deformation, constriction, rapid stretching, rapid compression, or pulse of high shear rate.

In some embodiments, shear force includes, but is not limited to, shear rate and other physical energy processes, such impact, cavitation, and turbulent mixing generated by a high energy fluid processor.

In some embodiments, the physical energy applied is shear rate, shear force, cavitation, impact, pressure, sonication, emulsion, or any other appropriate application method known in the art. In some aspects, the physical energy is applied via homogenization, microfluidization micro-emulsion, or French press. In some embodiments, the physical energy is shear force. In some embodiments, the shear force generates a shear rate. In some embodiments, the physical energy is pressure. In some embodiments, the physical energy is applied via homogenization or microfluidization. In some embodiments, the sonication is ultra-sonication.

Many different instruments that can apply physical energy are available, including high energy fluid processors, microfluidizers, French Press, high pressure homogenizers, bead mills, rotary blenders and rotor/stator devices. In some embodiments described herein, recombinant proteins may be solubilized using a microfluidizer. Microfluidizers may be purchased from commercial sources, such as the M110EH, M815, M700, LV1, LM10, LM20, M110Y or M110P Microfluidizers made by Microfluidics Corp (Westwood, MA).

A microfluidizer consists of an interchangeable fixed-geometry interaction chamber (such as G10Z, H10Z, H30Z, H210Z, L30Z, F20Y, or F12Y interaction chamber) in which flow is driven by a pump. In the Y geometry interaction chambers, the incoming flow is split into two or more streams and the recombined at high velocity to create steep velocity and pressure gradients, shear, cavitation, and heating. Fluid inside the interaction chamber experiences high flow velocities and a uniform application of shear forces, resulting in generation of a shear rate on the fluid. The intensity of homogenization can be altered by changing the geometry of the interaction chamber, altering the temperature, changing the pressure, or processing the same material through the instrument multiple times. There is also a complex interaction with the concentration of the incoming material, buffer composition, and the physiochemical properties of the solution, emulsion, or suspension. Several parameters may affect solubilization of the recombinant protein, including pressure, capillary diameter, temperature, number of homogenization passes, and buffer conditions. In addition, interaction chambers can have a single channel (single slot interaction chamber) or more than two channels (multi-slot interaction chamber). Multi-slot interaction chambers can be used to increase the volumetric flow rate through the interaction chamber, allowing for a greater volume of sample processing. Fluid flow rates inside a microfluidizer chamber can reach as much as 500 m/s through channels as small as 50 µm. Changes in the pressure result in changes in the shear rate as the fluids move through the interaction chamber.

Any appropriate homogenizer or microfluidizer known in art can be used. Microfluidizers and high pressure homogenizers are commercially available from a variety of vendors, including Microfluidics (Westwood MA), Thomas Scientific (Swedesboro, NJ), CAT Scientific (Paso Robles, CA), and Thermo Fisher Scientific. In some embodiments, the microfluidizer comprises a Z type interaction chamber. In some embodiments, the microfluidizer comprises a Y type interaction chamber. In some embodiments, the microfluidizer is an M-110P or LM10 microfluidizer.

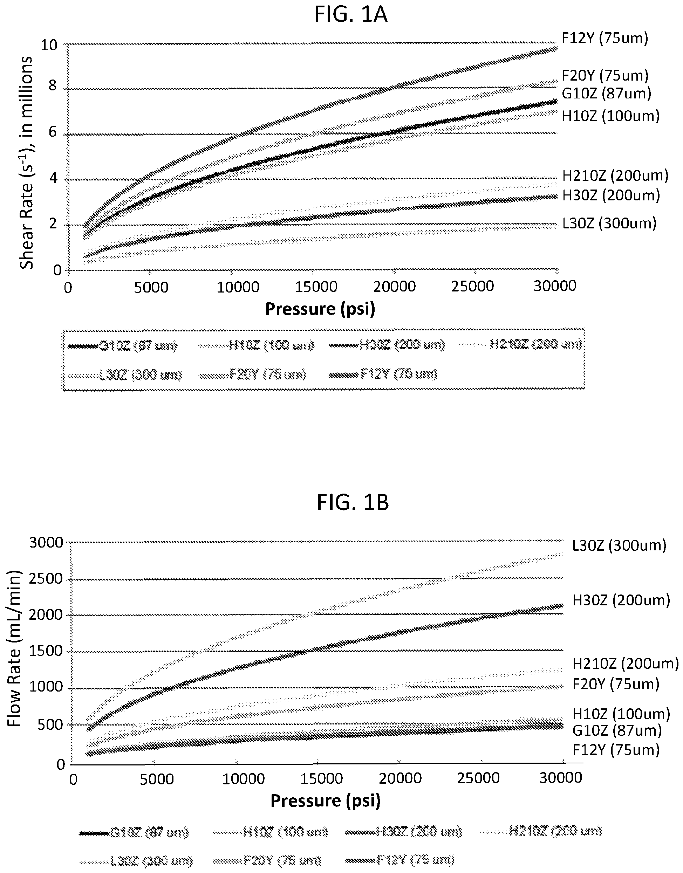

The shear rate generated by the specific combination of a microfluidizer interaction chamber and the pressure of the sample processing can be determined via information provided by the chamber manufacture. Examples of the calculated shear rate as a function of pressure in various single slotted Microfluidizer brand interaction chamber types is shown in **FIG. 1A****.** Flow rate as a function of pressure in various single slotted Microfluidizer brand interaction chamber types are shown in **FIG. 1B. FIG. 1A** and **1B** are adapted from the 2014 Microfluidics Processor User Guide produced by Microfluidics ^{™}. As shown in **FIG. 1A****,** the same amount of fluid pressure in two different chambers (e.g., the F12Y chamber and the L30Z chamber at 30,000 psi) result in almost an order of magnitude difference in the shear rates generated by the chambers, 10×10⁶s⁻¹ compared to 2×10⁶s⁻¹, respectively. Thus, changing the pressure of fluid flow through different interaction chambers results in different amounts of shear force and shear rates. And, the amount of shear rate can be changed and optimized by changing the fluid pressure in a chosen interaction chamber.

The application of shear force to a fluid or solution generates a fluid shear rate. In some embodiments, the shear force is applied via a microfluidizer. In some embodiments, the microfluidizer generates a shear rate. In some embodiments, the shear rate can be from between 1×10³ s⁻¹ to 1×10⁹ s⁻¹. The shear rate can be about 1×10³ s⁻¹, 1.5×10³ s⁻¹, 2×10³ s⁻¹, 2.5×10³ s⁻¹, 3×10³ s⁻¹, 3.5×10³ s⁻¹, 4×10³ s⁻¹, 4.5×10³ s⁻¹, 5×10³ s⁻¹, 5.5×10³ s⁻¹, 6×10³ s⁻¹, 6.5×10³ s⁻¹, 7×10³ s⁻¹, 7.5×10³ s⁻¹, 8×10³ s⁻¹, 8.5×10³ s⁻¹, 9×10³ s⁻¹, 9.5×10³ s⁻¹, 1×10⁴ s⁻¹, 1.5×10⁴ s⁻¹, 2×10⁴ s⁻¹, 2.5×10⁴ s⁻¹, 3×10⁴ s⁻¹, 3.5×10⁴ s⁻¹, 4×10⁴ s⁻¹, 4.5×10⁴ s⁻¹, 5×10⁴ s⁻¹, 5.5×10⁴ s⁻¹, 6×10⁴ s⁻¹, 6.5×10⁴ s⁻¹, 7×10⁴ s⁻¹, 7.5×10⁴ s⁻¹, 8×10⁴ s⁻¹, 8.5×10⁴ s⁻¹, 9×10⁴ s⁻¹, 9.5×10⁴ s⁻¹, 1×10⁵ s⁻¹, 1.5×10⁵ s⁻¹, 2×10⁵ s⁻¹, 2.5×10⁵ s⁻¹, 3×10⁵ s⁻¹, 3.5×10⁵ s⁻¹, 4×10⁵ s⁻¹, 4.5×10⁵ s⁻¹, 5×10⁵ s⁻¹, 5.5×10⁵ s⁻¹, 6×10⁵ s⁻¹, 6.5×10⁵ s⁻¹, 7×10⁵ s⁻¹, 7.5×10⁵ s⁻¹, 8×10⁵ s⁻¹, 8.5×10⁵ s⁻¹, 9×10⁵ s⁻¹, 9.5×10⁵ s⁻¹, 1×10⁶ s⁻¹, 1.5×10⁶ s⁻¹, 2×10⁶ s⁻¹, 2.5×10⁶ s⁻¹, 3×10⁶ s⁻¹, 3.5×10⁶ s⁻¹, 4×10⁶ s⁻¹, 4.5×10⁶ s⁻¹, 5×10⁶ s⁻¹, 5.5×10⁶ s⁻¹, 6×10⁶ s⁻¹, 6.5×10⁶ s⁻¹, 7×10⁶ s⁻¹, 7.5×10⁶ s⁻¹, 8×10⁶ s⁻¹, 8.5×10⁶ s⁻¹, 9×10⁶ s⁻¹, 9.5×10⁶ s⁻¹, 1×10⁷ s⁻¹, 1.5×10⁷ s⁻¹, 2×10⁷ s⁻¹, 2.5×10⁷ s⁻¹, 3×10⁷ s⁻¹, 3.5×10⁷ s⁻¹, 4×10⁷ s⁻¹, 4.5×10⁷ s⁻¹, 5×10⁷ s⁻¹, 5.5×10⁷ s⁻¹, 6×10⁷ s⁻¹, 6.5×10⁷ s⁻¹, 7×10⁷ s⁻¹, 7.5×10⁷ s⁻¹, 8×10⁷ s⁻¹, 8.5×10⁷ s⁻¹, 9×10⁷ s⁻¹, 9.5×10⁷ s⁻¹, 1×10⁸ s⁻¹, 1.5×10⁸ s⁻¹, 2×10⁸ s⁻¹, 2.5×10⁸ s⁻¹, 3×10⁸ s⁻¹, 3.5×10⁸ s⁻¹, 4×10⁸ s⁻¹, 4.5×10⁸ s⁻¹, 5×10⁸ s⁻¹, 5.5×10⁸ s⁻¹, 6×10⁸ s⁻¹, 6.5×10⁸ s⁻¹, 7×10⁸ s⁻¹, 7.5×10⁸ s⁻¹, 8×10⁸ s⁻¹, 8.5×10⁸ s⁻¹, 9×10⁸ s⁻¹, 9.5×10⁸ s⁻¹, 1×10⁹ s⁻¹. In some embodiments, the shear rate is about 6.5×10⁶ s⁻¹. In some embodiments, the shear rate is about 9.5×10⁶ s⁻¹.

The pressure can be from about 500 to 50,000 psi. The pressure can be at least about 500 psi, 750 psi, 1,000 psi, 2,000 psi, 3,000 psi, 4,000 psi, 5,000 psi, 10,000 psi, 15,000 psi, 20,000 psi, 25,000 psi, 20,000 psi, 25,000 psi, 40,000 psi, 45,000 psi, or 50,000 psi. The pressure can be between about 500 to 50,000 psi, 500 to 1,000 psi, 1,000 to 5,000 psi, 5,000 to 10,000 psi, 7,500 to 12,000 psi, 10,000 to 15,000 psi, 15,000 to 20,000 psi, 15,000-22,000, psi, 18,000-25,000 psi, 18,000-22,000 psi, 20,000 to 25,000 psi, 25,000 to 30,000 psi, 27,500 to 30,000 psi, 27,500 to 32,000 psi, 30,000 to 32,000 psi, 30,000 to 35,000 psi, 35,000 to 40,000 psi, 40,000 to 45,000 psi, or 45,000 to 50,000 psi. In some embodiments, the pressure is about 10,000 psi, 20,000 psi, 23,000 psi, or 30,000 psi. In some embodiments, the pressure is about 23,000 psi. In some embodiments, the pressure is about 30,000 psi. In one embodiment, the pressure is between 10,000 and 30,000 psi.

The spider silk protein can be processed via the physical energy at least once, i.e., the pressure, shear force and/or shear rate from the microfluidizer or homogenizer is applied to the spider silk protein once. In some embodiments, pressure, shear force and/or shear rate is applied once. The spider silk protein can also be processed via the physical energy more than once, i.e., pressure, shear force and/or shear rate is applied to the spider silk protein twice, three times, four times, or more. In some embodiments, the pressure, shear force and/or shear rate is applied three times. In some embodiments, the pressure, shear force and/or shear rate is applied two times.

The physical energy, i.e., the pressure, shear force and/or shear rate applied can be the same on each repeated pass or run. For example, a sample can be processed at 30,000 psi on the first pass, second, and third passes. In other embodiments, the pressure can be different on each pass or run. For example, a sample can be processed at 30,000 psi on the first pass, 23,000 psi on the second pass, and at 10,000 psi on the third pass. In another example, a sample can be processed with a shear rate of 6.5×10⁶ s⁻¹ on the first pass, 9.5×10⁶ s⁻¹ on the second pass, and at 5.5×10⁶ s⁻¹ on the third pass. In some embodiments, the pressure is the absolute pressure per square inch (psia). In some embodiments, the pressure is the gauge pressure per square inch (psig).

In some aspects, physical energy is sound energy applied via sonication. In such instances, the application of sound waves to a solution results in cavitation in the solution, resulting in the nucleation, growth, and collapse of bubbles in the solution that results in mechanical and physical deformation of the recombinant proteins in the solution, and thus increased solubility.

### Solvents and Buffer Conditions

The buffer conditions of the solution of the recombinant protein and insoluble cell portion, pellet, or lysate can also be altered to optimize homogenization or microfluidization and solubilization of the recombinant protein. Recombinant silk polypeptides form undesirable insoluble aggregates during production and purification due to their ability to aggregate and form β-sheet structures. Harsh chemical conditions are required for solubilization of these biological molecules, such as high molarity chaotrope solutions. Furthermore, the conditions required to re-solubilize the peptides during purification often degrade the proteins, resulting in poor yield and fibers with low tenacity and poor hand feel. However, the combination of a lower chaotrope concentration with the application of high physical energy, such as microfluidization or homogenization, can result in increased solubility and reduced degradation of the recombinant protein.

In some embodiments, solvents can be added to the insoluble cell portion, pellet, or lysate to solubilize the recombinant spider silk protein. Any appropriate solvent known in the art can be used, including but not limited to chaotropes and organic solvents. In some embodiments, the solvent is a chaotrope. Any appropriate chaotrope know in the art can be used, including, but not limited to, guanidine chloride (GdnHCl), guanidine thiocyanate (GdnSCN), guanidine isothiocyanate, n-butanol, ethanol, lithium perchlorate, lithium acetate, magnesium chloride, phenol, 2-propanol, sodium dodecyl sulfate, thiourea, and urea. In some embodiments, the solvent is guanidine chloride (GdnHCl). In some embodiments, the solvent is guanidine thiocyanate (GdnSCN). In some embodiments, the solvent is urea.

In some embodiments, the solvent is formulated in a water buffer. In some embodiments, the solvent is formulated in a 50 mM Tris, pH 7.5 buffer. Any appropriate buffer solution known in the art can be used to formulate the solvent, including, but not limited to, phosphate buffered saline (PBS) or the Good's buffers, such as Tris, Tricine, MES, PIPES, ACES, MOPS, MOPSO, TES, HEPES, TAPS, Bicine, TES, bis-tris propane, bis-tris methane, ADA, HEPBS, CHES, AMP, CAPS, CAPSO, glycinamide, glycylglycine, or any other appropriate buffer.

The solvent (e.g. achaotrope) can be added directly to the insoluble cell portion, pellet, or lysate, or added as a component of an aqueous buffer. The concentration of the solvent in the aqueous buffer can be altered as determined by one of skill in the art. In some embodiments, the concentration of the solvent in the aqueous buffer can be from between 0.01-10 M, 0.01-0.1 M, 0.1-0.5 M, 0.5-1 M, 1-2 M, 2-3 M, 3-4 M, 4-5 M, 5-6 M, 6-7 M, 7-8 M, 8-9 M, 9-10 M, or greater than 10 M. In some embodiments, the concentration of the solvent in the aqueous buffer can be at least about 0.1 M, 0.15 M, 0.2 M, 0.25 M, 0.3 M, 0.35 M, 0.4 M, 0.45 M, 0.5 M, 0.55 M, 0.6 M, 0.65 M, 0.7 M, 0.75 M, 0.8 M, 0.85 M, 0.9 M, 0.95 M, 1 M, 1.5 M, 2 M, 2.5 M, 3 M, 3.5 M, 4 M, 4.5 M, 5 M, 5.5 M, 6 M, 6.5 M, 7 M, 7.5 M, 8 M, 8.5 M, 9 M, 9.5 M, or 10 M, or greater.

In some embodiments, the solvent is added to the insoluble cell portion, pellet, or lysate at a specific mass to volume ratio. In such an embodiment, the total insoluble portion, pellet, or lysate mass is determined and a specific volume of a solution with a certain concentration of solvent or chaotrope is added. For example, a cell pellet weight is measured, and resuspended in a solution comprising a chaotropic agent such that the final volume of the cell mass is 15% of the total volume of the sample (e.g., the cell pellet is 0.75 mg, and resuspended in 4.25 mls of a buffer solution containing a 0.01-10M chaotrope solution). In another example, a cell pellet weight is measured, and resuspended in an equal volume of a solution comprising a solvent, resulting in a 50% cell mass to solvent volume ratio.

In some embodiments, the cell mass to solvent volume ratio can be from between 1-100%, 1-5%, 5-10%, 10-15%, 15-20%, 20-25%, 25-30%, 30-35%, 35-40%, 45-50%, 50-55%, 55-60%, 60-65%, 65-70%, 70-75%, 75-80%, 80-85%, 85-90%, 90-95%, or 95-100% cell mass. In some embodiments, the cell mass to solvent volume ratio can be at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% cell mass.

In some embodiments, the cell mass to solvent volume ratio can be from between 1-100%, 1-5%, 5-10%, 10-15%, 15-20%, 20-25%, 25-30%, 30-35%, 35-40%, 45-50%, 50-55%, 55-60%, 60-65%, 65-70%, 70-75%, 75-80%, 80-85%, 85-90%, 90-95%, or 95-100% solvent volume. In some embodiments, the cell mass to solvent volume ratio can be at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% solvent volume.

In some embodiments, the final concentration of the solvent in the solution comprising the aqueous buffer and the insoluble cell portion, pellet, or lysate can be from between 0.01-10 M, 0.01-0.1 M, 0.1-0.5 M, 0.5-1 M, 1-2 M, 2-3 M, 3-4 M, 4-5 M, 5-6 M, 6-7 M, 7-8 M, 8-9 M, or 9-10 M. In some embodiments, the final concentration of the solvent in the solution comprising the aqueous buffer and the cell lysate or pellet can be at least about 0.1 M, 0.15 M, 0.2 M, 0.25 M, 0.3 M, 0.35 M, 0.4 M, 0.45 M, 0.5 M, 0.55 M, 0.6 M, 0.65 M, 0.7 M, 0.75 M, 0.8 M, 0.85 M, 0.9 M, 0.95 M, 1 M, 1.5 M, 2 M, 2.5 M, 3 M, 3.5 M, 4 M, 4.5 M, 5 M, 5.5 M, 6 M, 6.5 M, 7 M, 7.5 M, 8 M, 8.5 M, 9 M, 9.5 M, or 10 M.

Additional buffer modifications may also be used, such as shear protectants, viscosity modifiers, and/or solutes that affect vesicle structural properties. Excipients may also be added to improve the efficiency of the homogenization or microfluidization such as membrane softening materials and molecular crowding agents. Other modifications to the buffer may include specific pH ranges and/or concentrations of salts, organic solvents, small molecules, detergents, zwitterions, amino acids, polymers, and/or any combination of the above including multiple concentrations.

In some embodiments, the insoluble cell portion, pellet, or lysate is incubated with the aqueous solution comprising a solvent. The amount of time the cell pellet or lysate is incubated with the solution can be altered. The incubation time can be from between 1 min to over 3 hours (180 min), 1 min to 60 min, 3 min to 90 min, 60 min to 120 min, 90 min to 150 min, or 120 min to 180 min. The incubation time can be at least 1 min, 5 min, 10 min, 15 min, 20 min, 30 min, 45 min, 60 min, 75 min, 90 min, 105 min, 120 min, 135 min, 150 min, 165 min, 180 min, or more. In some embodiments, the incubation time is 60 min. In some embodiments, the incubation time is 75 min. In some embodiments, the incubation time is 90 min. In some embodiments, the incubation time is 105 min. In some embodiments, the incubation time is 120 min.

The insoluble cell portion, pellet, or lysate can be incubated with the aqueous solution at 5-70°C. In some embodiments, the insoluble cell portion, pellet, or lysate is incubated with the aqueous solution at 5-10°C, 10-20°C, 10-15°C, 15-20°C, 20-30°C, 20-22°C, 20-25°C, 22-27°C, 25-27°C, 25-20°C, 27-30°C, 30-40°C, 40-50°C, 40-45°C, 45-50°C, 50-60°C, 50-55°C, 55-60°C, 60-70°C, 60-65°C, or 65-70°C. In some embodiments, the insoluble cell portion, pellet, or lysate is incubated with the aqueous solution at 20-30°C. In some embodiments, the insoluble cell portion, pellet, or lysate is incubated with the aqueous solution at 25°C. In some embodiments, the insoluble cell portion, pellet, or lysate is incubated with the aqueous solution at room temperature. In some embodiments, the insoluble cell portion, pellet, or lysate is incubated with the aqueous solution at no more than 30°C.

In some embodiments, the recombinant spider silk protein is expressed in the cytoplasm of a host cell. Isolation of the protein requires lysing the host cell to release the recombinant spider silk protein. Any appropriate method can be used to lyse the host cell, including, but not limited to, heat treatment, chemical treatment, shear disruption, physical homogenization, sonication, or chemical homogenization. Chemical treatment includes incubating the cells with chemicals or enzymes known to disrupt the plasma membrane of prokaryotic and eukaryotic cells, such as detergents, such as Triton X-100, Nonidet P-40, CHAPS, sodium dodecyl sulfate (SDS), or other appropriate detergents.

After lysing the cell, the insoluble portion comprising the recombinant spider silk protein can be collected by centrifuging the cell lysate, resulting in a cell pellet of insoluble material, including the recombinant spider silk protein. The centrifugation speed that pellets the insoluble recombinant protein can be determined by one of skill in the art. In some embodiments, the centrifuge speed is 100-10,000 × *g*. In some embodiments, the centrifuge speed is 100 × *g,* 200 × *g,* 300 × *g,* 400 × *g,* 500 × *g,* 600 × *g,* 700 × *g,* 800 × *g,* 900 × *g,* 1000 × *g,* 2000 × *g,* 3000 × *g,* 4000 × *g,* 5000 × *g,* 6000 × *g,* 7000 × *g,* 8000 × *g,* 9000 × *g,* or 10,000 × *g.*

In some instances, the insoluble cell portion or pellet can be resuspended or washed with a chaotrope, such as a urea solution, and then centrifuged again to produce a second cell pellet. This second cell pellet is then incubated with the aqueous solvent solution and subjected to the application of physical force to solubilize the recombinant spider silk protein. The chaotrope molarity in the wash solution can be from 0.1-10 M. In some embodiments, the chaotrope is urea. In some embodiments, the chaotrope is 4M urea.

In some embodiments, biological or chemical impurities of non-spider silk protein can be removed from the cell lysate or cell pellet. Removing impurities from the cell lysate or cell pellet can be accomplished by filtration, absorption (e.g. charcoal or solid-state absorption), dialysis and phase separation induced by coacervation or the use of various chemicals. In other embodiments, phase separation may be chemically induced by adding a cosmotrope and/or a compound used to precipitate the protein from solution.

In some embodiments, impurities are removed using filtration, microfiltration, diafiltration and/or ultrafiltration (e.g., against deionized water). Membranes suitable for microfiltration may include 0.1 uM to 1 uM. Non-limiting examples of suitable membranes for ultrafiltration include hydrophobic membranes (e.g., PES, PS, cellulose acetate) with molecular weight cut-offs of between 50 kDa and 800 kDa, 100 kDa and 800 kDa, 200 kDa and 800 kDa, 300 kDa and 800 kDa, 400 kDa and 800 kDa, 500 kDa and 800 kDa, 600 kDa and 800 kDa, 700 kDa and 800 kDa, 100 kDa and 700 kDa, 200 kDa and 700 kDa, 300 kDa and 700 kDa, 400 kDa and 700 kDa, 500 kDa and 700 kDa, 600 kDa and 700 kDa, or 500 kDa and 600 kDa. In some embodiments, ultrafiltration yields as retentate a recombinant protein slurry in water, and a permeate comprising the impurities. Suitable conditions for ultrafiltration (e.g., membranes, temperature, volume replacement) can be determined using methods known in the art geared towards maximizing permeate density. In some embodiments, the ultrafiltration provides a retentate that has a density of between 1 g/mL and 30 g/mL. In some embodiments, ultrafiltration comprises a concentrating step that yields a concentrated retentate, followed by a diafiltration step that removes the impurities and yields the suspended protein slurry in water. In some such embodiments, the concentrated retentate has a concentration factor of between 2-fold and 12-fold volume reduction to starting volume. In some embodiments, the diafiltration provides a constant volume replacement of between 3-fold and 10-fold.

Depending on the embodiment and the type of impurity to be removed, methods of removing impurities may differ. Removing lipid impurities from the isolated recombinant protein can be accomplished by methods known in the art. Non-limiting examples of such methods include absorption to charcoals or other absorption media that specifically bind lipids. Removing polysaccharide impurities from the isolated recombinant protein can be accomplished by methods known in the art. Non-limiting examples of such methods include treatment with enzymes that hydrolyze polysaccharides followed by removal of the small sugars produced by ultrafiltration. Non-limiting examples of such enzymes include glucanase, lyticase, mannase, and chitinase.

### Quantification

The isolated recombinant spider silk protein can be measured or quantified to assess the recovery (yield) and purity of the isolated protein. Any appropriate method may be used to measure or quantify the amount of isolated full length recombinant protein and recombinant protein fragments, including, but not limited so, size exclusion chromatography (SEC), Enzyme-Linked Immunosorbent Assay (ELISA), SDS-PAGE, Western blot (immunoblot), high performance liquid chromatography (HPLC), SEC-HPLC, liquid chromatography-mass spectrometry (LC-MS), or fast protein liquid chromatography (FPLC), or any other appropriate method known in the art, or any combination thereof. In one embodiment, the amount of full-length recombinant spider silk protein and recombinant protein fragments is measured using a Western blot. In another embodiment, the amount of full-length recombinant spider silk protein and recombinant protein fragments is measured using an Enzyme-Linked Immunosorbent Assay (ELISA). In another embodiment, the amount of full-length recombinant spider silk protein and recombinant protein fragments is measured using size exclusion chromatography (SEC).

In some embodiments, the isolated recombinant spider silk protein is at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, at least 99.9%, or at least 100% full-length recombinant spider silk protein, as measured by any appropriate method.

In some embodiments, the purity of the isolated recombinant spider silk protein is 5-10%, 10-15%, 15-20%, 20-25%, 25-30%, 30-35%, 35-40%, 45-50%, 50-55%, 55-60%, 60-65%, 65-70%, 70-75%, 75-80%, 80-85%, 85-90%, 90-95%, or 95-100%. In some embodiments, the purity of the isolated recombinant spider silk protein is at least 30%, at least 35%, at least 40%, at least 45%, at least 50%, at least 55%, at least 60%, at least 65%, at least 70%, at least 75%, at least 80%, at least 85 at least 90%, at least 91%, at least 92%, at least 93%, at least 94%, at least 95%, at least 96%, at least 97%, at least 98%, at least 99%, at least 99.5%, at least 99.9%, or at least 100%.

In some embodiments, the purity of the isolated recombinant spider silk protein is increased as compared to the starting insoluble material. For example, in an instance where the starting insoluble material is an insoluble or poorly soluble isolated recombinant spider silk protein or silk powder, the purity of the isolated recombinant spider silk protein can be increased via the solubilization and isolation methods described herein. In some embodiments, the purity of the isolated recombinant spider silk protein is increased 1-5%, 5-10%, 10-15%, 15-20%, 20-25%, 25-30%, 30-35%, 35-40%, 45-50%, 50-55%, 55-60%, 60-65%, 65-70%, 70-75%, 75-80%, 80-85%, 85-90%, 90-95%, or 95-100% as compared to the purity of the starting material. In some embodiments, the purity of the isolated recombinant spider silk protein is increased at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% as compared to the purity of the starting material. In some embodiments, the purity of the insoluble material is 0-99%, 1-5%, 5-10%, 10-15%, 15-20%, 20-25%, 25-30%, 30-35%, 35-40%, 45-50%, 50-55%, 55-60%, 60-65%, 65-70%, 70-75%, 75-80%, 80-85%, 85-90%, 90-95%, or 95-99.9%. In some embodiments, the purity of the insoluble material is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, at least 99.5%, or at least 99.9%.

In some embodiments, the isolated recombinant spider silk protein comprises at least 60%, at least 65%, at least 70%, at least 75%, at least 80%, at least 85%, at least 90%, or at least 95% full-length recombinant spider silk protein.

### Recombinant Spider Silk Compositions

US Patent 9,963,554, "Methods and Compositions for Synthesizing Improved Silk Fibers," incorporated herein by reference, discloses compositions for synthetic block copolymers, recombinant microorganisms for their production, and synthetic fibers comprising the proteins. US Patent Publication 2019/0100740, published April 4, 2019, "Modified Strains for the Production of Recombinant Silk," incorporated herein by reference, discloses engineered *Pichiapastoris* cells selected or genetically engineered to reduce degradation of recombinant proteins expressed by the yeast cells, and to methods of cultivating yeast cells for the production of useful compounds. Synthetic block copolymers and synthetic fibers comprising the proteins can also be produced in *Escherichia coli* by one of skill in the art.

Several types of native spider silks have been identified. The mechanical properties of each natively spun silk type are believed to be closely connected to the molecular composition of that silk. *See, e.g.,* Garb, J.E., et al., Untangling spider silk evolution with spidroin terminal domains, BMC Evol. Biol., 10:243 (2010); Bittencourt, D., et al., Protein families, natural history and biotechnological aspects of spider silk, Genet. Mol. Res., 11:3 (2012); Rising, A., et al., Spider silk proteins: recent advances in recombinant production, structure-function relationships and biomedical applications, Cell. Mol. Life Sci., 68:2, pg. 169-184 (2011); and Humenik, M., et al., Spider silk: understanding the structure-function relationship of a natural fiber, Prog. Mol. Biol. Transl. Sci., 103, pg. 131-85 (2011). For example:

Aciniform (AcSp) silks tend to have high toughness, a result of moderately high strength coupled with moderately high extensibility. AcSp silks are characterized by large block ("ensemble repeat") sizes that often incorporate motifs of poly serine and GPX. Tubuliform (TuSp or Cylindrical) silks tend to have large diameters, with modest strength and high extensibility. TuSp silks are characterized by their poly serine and poly threonine content, and short tracts of poly alanine. Major Ampullate (MaSp) silks tend to have high strength and modest extensibility. MaSp silks can be one of two subtypes: MaSp1 and MaSp2. MaSp1 silks are generally less extensible than MaSp2 silks, and are characterized by poly alanine, GX, and GGX motifs. MaSp2 silks are characterized by poly alanine, GGX, and GPX motifs. Minor Ampullate (MiSp) silks tend to have modest strength and modest extensibility. MiSp silks are characterized by GGX, GA, and poly A motifs, and often contain spacer elements of approximately 100 amino acids. Flagelliform (Flag) silks tend to have very high extensibility and modest strength. Flag silks are usually characterized by GPG, GGX, and short spacer motifs.

The properties of each silk type can vary from species to species, and spiders leading distinct lifestyles (e.g. sedentary web spinners vs. vagabond hunters) or that are evolutionarily older may produce silks that differ in properties from the above descriptions (for descriptions of spider diversity and classification, see Hormiga, G., and Griswold, C.E., Systematics, phylogeny, and evolution of orb-weaving spiders, Annu. Rev. Entomol. 59, pg. 487-512 (2014); and Blackedge, T.A. et al., Reconstructing web evolution and spider diversification in the molecular era, Proc. Natl. Acad. Sci. U.S.A., 106:13, pg. 5229-5234 (2009)). However, synthetic block copolymer polypeptides having sequence similarity and/or amino acid composition similarity to the repeat domains of native silk proteins can be used to manufacture on commercial scales consistent silk-like fibers that recapitulate the properties of corresponding natural silk fibers.

In some embodiments, the recombinant spider silks are a highly crystalline silk protein, a high beta sheet content silk protein, or a low solubility silk protein. In some embodiments, the recombinant spider silk protein has a solubility threshold of less than 95, 90%, 85%, 80%, 75%, 70%, 65%, 60%, 55%, 50%, 45%, 40%, 35%, 30%, 25%, 20%, 10%, or 5% in a non-chaotropic solvent. In some embodiments, the solubility threshold is the amount of protein that is soluble in a non-chaotropic solvent after centrifugation.

### Silk Nucleotide and Peptide Sequences

A list of putative silk sequences can be compiled by searching GenBank for relevant terms, e.g. "spidroin" "fibroin" "MaSp", and those sequences can be pooled with additional sequences obtained through independent sequencing efforts. Sequences are then translated into amino acids, filtered for duplicate entries, and manually split into domains (NTD, REP, CTD). In some embodiments, candidate amino acid sequences are reverse translated into a DNA sequence optimized for expression in *Pichia (Komagataella) pastoris.* The DNA sequences are each cloned into an expression vector and transformed into *Pichia (Komagataella) pastoris.* In some embodiments, various silk domains demonstrating successful expression and secretion are subsequently assembled in combinatorial fashion to build silk molecules capable of fiber formation.

Silk polypeptides are characteristically composed of a repeat domain (REP) flanked by non-repetitive regions (e.g., C-terminal and N-terminal domains). The repeat domain exhibits a hierarchical architecture. The repeat domain comprises a series of blocks (also called repeat units). The blocks are repeated, sometimes perfectly and sometimes imperfectly (making up a quasi-repeat domain), throughout the silk repeat domain. The length and composition of blocks varies among different silk types and across different species. **Table 1** lists examples of block sequences from selected species and silk types, with further examples presented in Rising, A. et al., Spider silk proteins: recent advances in recombinant production, structure-function relationships and biomedical applications, Cell Mol. Life Sci., 68:2, pg 169-184 (2011); and Gatesy, J. et al., Extreme diversity, conservation, and convergence of spider silk fibroin sequences, Science, 291:5513, pg. 2603-2605 (2001). In some cases, blocks may be arranged in a regular pattern, forming larger macro-repeats that appear multiple times (usually 2-8) in the repeat domain of the silk sequence. Repeated blocks inside a repeat domain or macro-repeat, and repeated macro-repeats within the repeat domain, may be separated by spacing elements. Block sequences may comprise a glycine rich region followed by a poly A region. Short (-1-10) amino acid motifs may appear multiple times inside of blocks. A subset of commonly observed motifs is depicted in Figure 1. Blocks from different natural silk polypeptides can be selected without reference to circular permutation (i.e., identified blocks that are otherwise similar between silk polypeptides may not align due to circular permutation). Thus, for example, a "block" of SGAGG (SEQ ID NO: 39) is, for the purposes of the methods and compositions described herein, the same as GSGAG (SEQ ID NO: 40) and the same as GGSGA (SEQ ID NO: 41); they are all just circular permutations of each other. The particular permutation selected for a given silk sequence can be dictated by convenience (usually starting with a G) more than anything else. Silk sequences obtained from the NCBI database can be partitioned into blocks and non-repetitive regions.

**Table 1: Samples of Block Sequences**

| **Species** | **Silk Type** | **SEQ ID NO** | **Representative Block Amino Acid Sequence** |
|---|---|---|---|
| *Aliatypus gulosus* | Fibroin 1 | 1 | GAASSSSTIITTKSASASAAADASAAATASAASRSSANAAASAFAQSFSS ILLESGYFCSIFGSSISSSYAAAIASAASRAAAESNGYTTHAYACAKAVA SAVERVTSGADAYAYAQAISDALSHALLYTGRLNTANANSLASAFAYAFA NAAAQASASSASAGAASASGAASASGAGSAS |
| *Plectreurys tristis* | Fibroin 1 | 2 | GAGAGAGAGAGAGAGAGSGASTSVSTSSSSGSGAGAGAGSGAGSGAGAGS GAGAGAGAGGAGAGFGSGLGLGYGVGLSSAQAQAQAQAAAQAQAQAQA YAAAQAQAQAQAQAQAAAAAAAAAAA |
| *Plectreurys tristis* | Fibroin 4 | 3 | GAAQKQPSGESSVATASAAATSVTSGGAPVGKPGVPAPIFYPQGPLQQGP APGPSNVQPGTSQQGPIGGVGGSNAFSSSFASALSLNRGFTEVISSASAT AVASAFQKGLAPYGTAFALSAASAAADAYNSIGSGANAFAYAQAFARVLY PLVQQYGLSSSAKASAFASAIASSFSSGTSGQGPSIGQQQPPVTISAASA SAGASAAAVGGGQVGQGPYGGQQQSTAASASAAAATATS |
| *Araneus gemmoides* | TuSp | 4 | GNVGYQLGLKVANSLGLGNAQALASSLSQAVSAVGVGASSNAYANAVSNA VGQVLAGQGILNAANAGSLASSFASALSSSAASVASQSASQSQAASQSQA AASAFRQAASQSASQSDSRAGSQSSTKTTSTSTSGSQADSRSASSSASQA SASAFAQQSSASLSSSSSFSSAFSSATSISAV |
| *Argiope aurantia* | TuSp | 5 | GSLASSFASALSASAASVASSAAAQAASQSQAAASAFSRAASQSASQSAA RSGAQSISTTTTTSTAGSQAASQSASSAASQASASSFARASSASLAASSS FSSAFSSANSLSALGNVGYQLGFNVANNLGIGNAAGLGNALSQAVSSVGV GASSSTYANAVSNAVGQFLAGQGILNAANA |
| *Deinopis spinosa* | TuSp | 6 | GASASAYASAISNAVGPYLYGLGLFNQANAASFASSFASAVSSAVASASA SAASSAYAQSAAAQAQAASSAFSQAAAQSAAAASAGASAGAGASAGAGAV AGAGAVAGAGAVAGASAAAASQAAASSSASAVASAFAQSASYALASSSAF ANAFASATSAGYLGSLAYQLGLTTAYNLGLSNAQAFASTLSQAVTGVGL |
| *Nephila clavipes* | TuSp | 7 | GATAASYGNALSTAAAQFFATAGLLNAGNASALASSFARAFSASAESQSF AQSQAFQQASAFQQAASRSASQSAAEAGSTSSSTTTTTSAARSQAASQSA SSSYSSAFAQAASSSLATSSALSRAFSSVSSASAASSLAYSIGLSAARSL GIADAAGLAGVLARAAGALGQ |
| *Argiope trifasciata* | Flag | 8 | GGAPGGGPGGAGPGGAGFGPGGGAGFGPGGGAGFGPGGAAGGPGGPGGPG GPGGAGGYGPGGAGGYGPGGVGPGGAGGYGPGGAGGYGPGGSGPGGAGPG GAGGEGPVTVDVDVTVGPEGVGGGPGGAGPGGAGFGPGGGAGFGPGGAPG APGGPGGPGGPGGPGGPGGVGPGGAGGYGPGGAGGVGPAGTGGFGPGGAG GFGPGGAGGFGPGGAGGFGPAGAGGYGPGGVGPGGAGGFGPGGVGPGGSG PGGAGGEGPVTVDVDVSV |
| *Nephila clavipes* | Flag | 9 | GVSYGPGGAGGPYGPGGPYGPGGEGPGGAGGPYGPGGVGPGGSGPGGYGP GGAGPGGYGPGGSGPGGYGPGGSGPGGYGPGGSGPGGYGPGGSGPGGYGP GGYGPGGSGPGGSGPGGSGPGGYGPGGTGPGGSGPGGYGPGGSGPGGSGP GGYGPGGSGPGGFGPGGSGPGGYGPGGSGPGGAGPGGVGPGGFGPGGAGP GGAAPGGAGPGGAGPGGAGPGGAGPGGAGPGGAGPGGAGGAGGAGGSGGA GGSGGTTIIEDLDITIDGADGPITISEELPISGAGGSGPGGAGPGGVGPG GSGPGGVGPGGSGPGGVGPGGSGPGGVGPGGAGGPYGPGGSGPGGAGGAG GPGGAYGPGGSYGPGGSGGPGGAGGPYGPGGEGPGGAGGPYGPGGAGGPY GPGGAGGPYGPGGEGGPYGP |
| *Latrodectus hesperus* | AcSp | 10 | GINVDSDIGSVTSLILSGSTLQMTIPAGGDDLSGGYPGGFPAGAQPSGGA PVDFGGPSAGGDVAAKLARSLASTLASSGVFRAAFNSRVSTPVAVQLTDA LVQKIASNLGLDYATASKLRKASQAVSKVRMGSDTNAYALAISSALAEVL SSSGKVADANINQIAPQLASGIVLGVSTTAPQFGVDLSSINVNLDISNVA RNMQASIQGGPAPITAEGPDFGAGYPGGAPTDLSGLDMGAPSDGSRGGDA TAKLLQALVPALLKSDVFRAIYKRGTRKQVVQYVTNSALQQAASSLGLDA STISQLQTKATQALSSVSADSDSTAYAKAFGLAIAQVLGTSGQVNDANVN QIGAKLATGILRGSSAVAPRLGIDLS |
| *Argiope trifasciata* | AcSp | 11 | GAGYTGPSGPSTGPSGYPGPLGGGAPFGQSGFGGSAGPQGGFGATGGASA GLISRVANALANTSTLRTVLRTGVSQQIASSVVQRAAQSLASTLGVDGNN LARFAVQAVSRLPAGSDTSAYAQAFSSALFNAGVLNASNIDTLGSRVLSA LLNGVSSAAQGLGINVDSGSVQSDISSSSSFLSTSSSSASYSQASASSTS |
| *Uloborus diversus* | AcSp | 12 | GASAADIATAIAASVATSLQSNGVLTASNVSQLSNQLASYVSSGLSSTAS SLGIQLGASLGAGFGASAGLSASTDISSSVEATSASTLSSSASSTSVVSS INAQLVPALAQTAVLNAAFSNINTQNAIRIAELLTQQVGRQYGLSGSDVA TASSQIRSALYSVQQGSASSAYVSAIVGPLITALSSRGVVNASNSSQIAS SLATAILQFTANVAPQFGISIPTSAVQSDLSTISQSLTAISSQTSSSVDS STSAFGGISGPSGPSPYGPQPSGPTFGPGPSLSGLTGFTATFASSFKSTL ASSTQFQLIAQSNLDVQTRSSLISKVLINALSSLGISASVASSIAASSSQ SLLSVSA |
| *Euprosthenop s australis* | MaSp1 | 13 | GGQGGQGQGRYGQGAGSSAAAAAAAAAAAAAA |
| *Tetragnatha kauaiensis* | MaSp1 | 14 | GGLGGGQGAGQGGQQGAGQGGYGSGLGGAGQGASAAAAAAAA |
| *Argiope aurantia* | MaSp2 | 15 | GGYGPGAGQQGPGSQGPGSGGQQGPGGLGPYGPSAAAAAAAA |
| *Deinopis spinosa* | MaSp2 | 16 | GPGGYGGPGQQGPGQGQYGPGTGQQGQGPSGQQGPAGAAAAAAAAA |
| *Nephila clavata* | MaSp2 | 17 | GPGGYGLGQQGPGQQGPGQQGPAGYGPSGLSGPGGAAAAAAA |
| *Deinopis Spinosa* | MiSp | 18 | GAGYGAGAGAGGGAGAGTGYGGGAGYGTGSGAGYGAGVGYGAGAGAGGGA GAGAGGGTGAGAGGGAGAGYGAGTGYGAGAGAGGGAGAGAGAGAGAGA GSGAGAGYGAGAGYGAGAGAGGVAGAGAAGGAGAAGGAGAAGGAGAAGGA GAGAGAGSGAGAGAGGGARAGAGG |
| *Latrodectus hesperus* | MiSp | 19 | GGGYGRGQGAGAGVGAGAGAAAGAAAIARAGGYGQGAGGYGQGQGAGAAA GAAAGAGAGGYGQGAGGYGRGQGAGAGAGAGAGARGYGQGAGAGAAAGAA ASAGAGGYGQGAGGYGQGQGAGAAAGAAASAGAGGYGQGAGGYGQGQGA |
| *Nephila clavipes* | MiSp | 20 | GAGAGGAGYGRGAGAGAGAAAGAGAGAAAGAGAGAGGYGGQGGYGAGAGA GAAAAAGAGAGGAAGYSRGGRAGAAGAGAGAAAGAGAGAGGYGGQGGYGA GAGAGAAAAAGAGSGGAGGYGRGAGAGAAAGAGAAAGAGAGAGGYGGQGG YGAGAGAAAAA |
| *Nephilengys cruentata* | MiSp | 21 | GAGAGVGGAGGYGSGAGAGAGAGAGAASGAAAGAAAGAGAGGAGGYGTGQ GYGAGAGAGAGAGAGGAGGYGRGAGAGAGAGAGGAGGYGAGQGYGAGAGA GAAAAAGDGAGAGGAGGYGRGAGAGAGAGAAAGAGAGGAGGYGAGQGYGA GAGAGAAAGAGAGGAGGYGAGQGYGAGAGAGAAAAA |
| *Uloborus diversus* | MiSp | 22 | GSGAGAGSGYGAGAGAGAGSGYGAGSSASAGSAINTQTVTSSTTTSSQSS AAATGAGYGTGAGTGASAGAAASGAGAGYGGQAGYGQGAGASARAAGSGY GAGAGAAAAAGSGYGAGAGAGAGSGYGAGAAA |
| *Uloborus diversus* | MiSp | 23 | GAGAGYRGQAGYIQGAGASAGAAAAGAGVGYGGQAGYGQGAGASAGAAAA AGAGAGRQAGYGQGAGASAGAAAAGAGAGRQAGYGQGAGASAGAAAAGAD AGYGGQAGYGQGAGASAGAAASGAGAGYGGQAGYGQGAGASAGAAAAGAG AGYLGQAGYGQGAGASAGAAAGAGAGYGGQAGYGQGTGAAASAAASSA |
| *Araneus ventricosus* | MaSp1 | 24 | GGQGGQGGYGGLGSQGAGQGGYGAGQGAAAAAAAAGGAGGAGRGGLGAGG AGQGYGAGLGGQGGAGQAAAAAAAGGAGGARQGGLGAGGAGQGYGAGLGG QGGAGQGGAAAAAAAAGGQGGQGGYGGLGSQGAGQGGYGAGQGGAAAAAA AAGGQGGQGGYGGLGSQGAGQGGYGGRQGGAGAAAAAAAA |
| *Dolomedes tenebrosus* | MaSp1 | 25 | GGAGAGQGSYGGQGGYGQGGAGAATATAAAAGGAGSGQGGYGGQGGLGGY GQGAGAGAAAAAAAAAGGAGAGQGGYGGQGGQGGYGQGAGAGAAAAAAGG AGAGQGGYGGQGGYGQGGGAGAAAAAAAASGGSGSGQGGYGGQGGLGGYG QGAGAGAGAAASAAAA |
| *Nephilengys cruentata* | MaSp | 26 | GGAGQGGYGGLGGQGAGAAAAAAGGAGQGGYGGQGAGQGAAAAAASGAGQ GGYEGPGAGQGAGAAAAAAGGAGQGGYGGLGGQGAGQGAGAAAAAAGGAG QGGYGGLGGQGAGQGAGAAAAAAGGAGQGGYGGQGAGQGAAAAAAGGAGQ GGYGGLGSGQGGYGRQGAGAAAAAAAA |
| *Nephilengys cruentata* | MaSp | 27 | GGAGQGGYGGLGGQGAGAAAAAAGGAGQGGYGGQGAGQGAAAAAASGAGQ GGYGGPGAGQGAGAAAAAAGGAGQGGYGGLGGQGAGQGAGAAAAAAGGAG QGGYGGQGAGQGAAAAAAGGAGQGGYGGLGSGQGGYGGQGAGAAAAAGGA GQGGYGGLGGQGAGQGAGAAAAAA |

Fiber-forming block copolymer polypeptides from the blocks and/or macro-repeat domains, according to certain embodiments, is described in International Publication No. WO/2015/042164, incorporated by reference. Natural silk sequences obtained from a protein database such as GenBank or through *de novo* sequencing are broken up by domain (N-terminal domain, repeat domain, and C-terminal domain). The N-terminal domain and C-terminal domain sequences selected for the purpose of synthesis and assembly into fibers include natural amino acid sequence information and other modifications described herein. The repeat domain is decomposed into repeat sequences containing representative blocks, usually 1-8 depending upon the type of silk, that capture critical amino acid information while reducing the size of the DNA encoding the amino acids into a readily synthesizable fragment. In some embodiments, a properly formed block copolymer polypeptide comprises at least one repeat domain comprising at least 1 repeat sequence, and is optionally flanked by an N-terminal domain and/or a C-terminal domain.

In some embodiments, a repeat domain comprises at least one repeat sequence. In some embodiments, the repeat sequence is 150-300 amino acid residues. In some embodiments, the repeat sequence comprises a plurality of blocks. In some embodiments, the repeat sequence comprises a plurality of macro-repeats. In some embodiments, a block or a macro-repeat is split across multiple repeat sequences.

In some embodiments, the repeat sequence starts with a Glycine, and cannot end with phenylalanine (F), tyrosine (Y), tryptophan (W), cysteine (C), histidine (H), asparagine (N), methionine (M), or aspartic acid (D) to satisfy DNA assembly requirements. In some embodiments, some of the repeat sequences can be altered as compared to native sequences. In some embodiments, the repeat sequences can be altered such as by addition of a serine to the C terminus of the polypeptide (to avoid terminating in F, Y, W, C, H, N, M, or D). In some embodiments, the repeat sequence can be modified by filling in an incomplete block with homologous sequence from another block. In some embodiments, the repeat sequence can be modified by rearranging the order of blocks or macrorepeats.

In some embodiments, non-repetitive N- and C-terminal domains can be selected for synthesis. In some embodiments, N-terminal domains can be by removal of the leading signal sequence, e.g., as identified by SignalP (Peterson, T.N., et. Al., SignalP 4.0: discriminating signal peptides from transmembrane regions, Nat. Methods, 8:10, pg. 785-786 (2011).

In some embodiments, the N-terminal domain, repeat sequence, or C-terminal domain sequences can be derived from *Agelenopsis aperta, Aliatypus gulosus, Aphonopelma seemanni, Aptostichus sp. AS217, Aptostichus* sp. AS220, *Araneus diadematus, Araneus gemmoides, Araneus ventricosus, Argiope amoena, Argiope argentata, Argiope bruennichi, Argiope trifasciata, Atypoides riversi, Avicularia juruensis, Bothriocyrtum californicum, Deinopis Spinosa, Diguetia canities, Dolomedes tenebrosus, Euagrus chisoseus, Euprosthenops australis, Gasteracantha mammosa, Hypochilus thorelli, Kukulcania hibernalis, Latrodectus hesperus, Megahexura fulva, Metepeira grandiosa, Nephila antipodiana, Nephila clavata, Nephila clavipes, Nephila madagascariensis, Nephila pilipes, Nephilengys cruentata, Parawixia bistriata, Peucetia viridans, Plectreurys tristis, Poecilotheria regalis, Tetragnatha kauaiensis, or Uloborus diversus.*

In some embodiments, the silk polypeptide nucleotide coding sequence can be operatively linked to an alpha mating factor nucleotide coding sequence. In some embodiments, the silk polypeptide nucleotide coding sequence can be operatively linked to another endogenous or heterologous secretion signal coding sequence. In some embodiments, the silk polypeptide nucleotide coding sequence can be operatively linked to a 3X FLAG nucleotide coding sequence. In some embodiments, the silk polypeptide nucleotide coding sequence is operatively linked to other affinity tags such as a 6-8 His residues (SEQ ID NO: 42) nucleotide coding sequence.

### Secretion Signals

The amount of protein that is secreted from a cell varies significantly between proteins, and is dependent in part on the secretion signal that is operably linked to the protein in its nascent state. A number of secretion signals are known in the art, and some are commonly used for production of secreted recombinant proteins. Prominent among these is the secretion signal of the α-mating factor (αMF) of *Saccharomyces cerevisiae,* which consists of a N-terminal 19-amino-acid signal peptide (also referred to herein as pre-αMF(sc)) followed by a 70-amino-acid leader peptide (also referred to herein as pro-αMF(sc)). Inclusion of pro-αMF(sc) in the secretion signal of the αMF of *Saccharomyces cerevisiae* (also referred to herein as pre-αMF(sc) / pro-αMF(sc) has proven critical for achieving high secreted yields of proteins. Addition of pro-αMF(sc) or functional variants thereof to signal peptides other than pre-αMF(sc) has also been explored as a means of achieving secretion of recombinant proteins, but has shown variable degrees of effectiveness, increasing secretion for certain recombinant proteins in certain recombinant host cells but having no effect or decreasing secretion for other recombinant proteins.

The use of multiple distinct secretion signals can improve the secreted yields of recombinant proteins, as described in US Application 15/724,196. Compared to recombinant host cells that comprise multiple polynucleotide sequences encoding a recombinant protein operably linked to just one secretion signal (e.g., pre-αMF(sc) / pro-αMF(sc)), recombinant host cells that comprise the same number of polynucleotide sequences encoding the recombinant protein operably linked to at least 2 distinct secretion signals produce higher secreted yields of the recombinant protein. Without wishing to be bound by theory, the use of at least 2 distinct secretion signals may permit the recombinant host cell to engage distinct cellular secretory pathways to effect efficient secretion of the recombinant protein and thus prevent over-saturation of any one secretion pathway.

At least one of the distinct secretion signals comprises a signal peptide may be selected from Table 2 or 3 or is a functional variant that has an at least 80% amino acid sequence identity to a signal peptide selected from Table 2 or 3. In some embodiments, the functional variant is a signal peptide selected from Table 2 or 3 that comprises one or two substituted amino acids. In some such embodiments, the functional variant has an at least 85%, at least 90%, at least 95%, or at least 99% amino acid sequence identity to a signal peptide selected from Table 2 or 3. In some embodiments, the signal peptide mediates translocation of the nascent recombinant protein into the ER post-translationally (i.e., protein synthesis precedes translocation such that the nascent recombinant protein is present in the cell cytosol prior to translocating into the ER). In other embodiments, the signal peptide mediates translocation of the nascent recombinant protein into the ER co-translationally (i.e., protein synthesis and translocation into the ER occur simultaneously). An advantage of using a signal peptide that mediates co-translational translocation into the ER is that recombinant proteins prone to rapid folding are prevented from assuming conformations that hinder translocation into the ER and thus secretion.

**Table 2 - Secretion Signals**

| **Source Gene ID** | **Species** | **Name** | **SEQ ID NO** | **Sequence** |
|---|---|---|---|---|
| PEP4 | *Saccharomyces cerevisiae* | pre-PEP4(sc) | 28 | MFSLKALLPLALLLVSANQVAA |
| PAS_chr1-1_0130 | *Pichia pastoris* | pre-DSE4(pp) | 29 | MSFSSNVPQLFLLLVLLTNIVSG |
| PAS_chr3_0076 | *Pichia pastoris* | pre-EPXl(pp) | 30 | MKLSTNLILAIAAASAVVSA |
| P00698 | *Gallus gallus* | pre-CLSP(gg) | 31 | MRSLLILVLCFLPLAALG |

**Table 3 - Recombinant Secretion Signals**

| **Name** | **SEQ ID NO** | **Sequence** |
|---|---|---|
| pre-αMF(sc) / pro-αMF(sc) | 32 | MRFPSIFTAVLFAASSALAAPVNTTTEDETAQIPAEAVIGYLDLEGDFDV AVLPFSNSTNNGLLFINTTIASIAAKEEGVSLDKREAEA |
| pre-αMF(sc) / *pro-αMF(sc) | 33 | MRFPSIFTAVLFAASSALAAPVNTTTEDETAQIPAEAVIGYSDLEGDFDV AVLPFSNSTNNGLLFINTTIASIAAKEEGVSLEKREAEA |
| pre-PEP4(sc) / *pro-αMF(sc) | 34 | MFSLKALLPLALLLVSANQVAAAPVNTTTEDETAQIPAEAVIGYSDLEGD FDVAVLPFSNSTNNGLLFINTTIASIAAKEEGVSLEKREAEA |
| pre-DSE4(pp) / pro-αMF(sc) | 35 | MSFSSNVPQLFLLLVLLTNIVSGAPVNTTTEDETAQIPAEAVIGYSDLEG DFDVAVLPFSNSTNNGLLFINTTIASIAAKEEGVSLEKREAEA |
| pre-EPXl(pp) / *pro-αMF(sc) | 36 | MKLSTNLILAIAAASAVVSAAPVNTTTEDETAQIPAEAVIGYSDLEGDFD VAVLPFSNSTNNGLLFINTTIASIAAKEEGVSLEKREAEA |
| pre-CLSP(gg) / *pro-αMF(sc) | 37 | MRSLLILVLCFLPLAALGAPVNTTTEDETAQIPAEAVIGYSDLEGDFDVA VLPFSNSTNNGLLFINTTIASIAAKEEGVSLEKREAEA |

### Expression Vectors

The expression vectors described herein can be produced following the teachings of the present specification in view of techniques known in the art. Sequences, for example vector sequences or sequences encoding transgenes, can be commercially obtained from companies such as Integrated DNA Technologies, Coralville, IA or DNA 2.0, Menlo Park, CA. Exemplified herein are expression vectors that direct high-level expression of the chimeric silk polypeptides.

Another standard source for the polynucleotides described herein is polynucleotides isolated from an organism (e.g., bacteria), a cell, or selected tissue. Nucleic acids from the selected source can be isolated by standard procedures, which typically include successive phenol and phenol/chloroform extractions followed by ethanol precipitation. After precipitation, the polynucleotides can be treated with a restriction endonuclease which cleaves the nucleic acid molecules into fragments. Fragments of the selected size can be separated by a number of techniques, including agarose or polyacrylamide gel electrophoresis or pulse field gel electrophoresis (Care et al. (1984) Nuc. Acid Res. 12:5647-5664; Chu et al. (1986) Science 234:1582; Smith et al. (1987) Methods in Enzymology 151:461), to provide an appropriate size starting material for cloning.

Another method of obtaining the nucleotide components of the expression vectors or constructs is PCR. General procedures for PCR are taught in MacPherson et al., PCR: A PRACTICAL APPROACH, (IRL Press at Oxford University Press, (1991)). PCR conditions for each application reaction may be empirically determined. A number of parameters influence the success of a reaction. Among these parameters are annealing temperature and time, extension time, Mg2+ and ATP concentration, pH, and the relative concentration of primers, templates and deoxyribonucleotides. Exemplary primers are described below in the Examples. After amplification, the resulting fragments can be detected by agarose gel electrophoresis followed by visualization with ethidium bromide staining and ultraviolet illumination.

Another method for obtaining polynucleotides is by enzymatic digestion. For example, nucleotide sequences can be generated by digestion of appropriate vectors with suitable recognition restriction enzymes. Restriction cleaved fragments may be blunt ended by treating with the large fragment of *E. coli* DNA polymerase I (Klenow) in the presence of the four deoxynucleotide triphosphates (dNTPs) using standard techniques.

Polynucleotides are inserted into suitable backbones, for example, plasmids, using methods well known in the art. For example, insert and vector DNA can be contacted, under suitable conditions, with a restriction enzyme to create complementary or blunt ends on each molecule that can pair with each other and be joined with a ligase. Alternatively, synthetic nucleic acid linkers can be ligated to the termini of a polynucleotide. These synthetic linkers can contain nucleic acid sequences that correspond to a particular restriction site in the vector DNA. Other means are known and available in the art. A variety of sources can be used for the component polynucleotides.

In some embodiments, expression vectors containing an R, N, or C sequence are transformed into a host organism for expression and/or secretion. In some embodiments, the expression vectors comprise a secretion signal. In some embodiments, the expression vector comprises a terminator signal. In some embodiments, the expression vector is designed to integrate into a host cell genome and comprises: regions of homology to the target genome, a promoter, a secretion signal, a tag (e.g., a Flag tag), a termination/polyA signal, a selectable marker for *Pichia,* a selectable marker for *E. coli,* an origin of replication for *E. coli,* and restriction sites to release fragments of interest.

The vectors of the present invention can further comprise targeting sequences that direct integration of the spider silk protein coding sequences to specific locations in the genome of host cells. Non-limiting examples of such targeting sequences include nucleotide sequences that are identical to nucleotide sequences present in the genome of a host cell. In some embodiments, the targeting sequences are identical to repetitive elements in the genome of host cells. In some embodiments, the targeting sequences are identical to transposable elements in the genome of host cells.

In some embodiments, recombinant host cells are provided herein that comprise the vectors described herein. In some embodiments, the vectors are stably integrated within the genome (e.g., a chromosome) of the recombinant host cells, e.g., via homologous recombination or targeted integration. Non-limiting examples of suitable sites for genomic integration include the Ty1 loci in the *Saccharomyces cerevisiae* genome, the rDNA and HSP82 loci in the *Pichiapastoris* genome, and transposable elements that have copies scattered throughout the genome of the recombinant host cells. In other embodiments, the vectors are not stably integrated within the genome of the recombinant host cells but rather are extrachromosomal.

### Host Cell Transformants

Host cells transformed with nucleic acid molecules or vectors that express spider silk polypeptides, and descendants thereof, are provided. These cells can also carry the nucleic acid sequences on vectors, which may but need not be freely replicating vectors. In other embodiments, the nucleic acids have been integrated into the genome of the host cells.

In some embodiments, microorganisms or host cells that enable the large-scale production of block copolymer polypeptides include a combination of: 1) the ability to produce large (>50kDa) polypeptides, 2) resistance to contaminants (such as viruses and bacterial contaminations) at large-scale, and 3) the existing know-how for growing and processing the organism is large-scale (1-2000m³) bioreactors.

In some embodiments, the host cell expresses the recombinant spider silk protein intracellularly and the protein remains in the host cell. In some embodiments, the host cell expresses the recombinant spider silk protein intracellularly and the protein is secreted.

A variety of host organisms can be engineered/transformed to comprise a block copolymer polypeptide expression system. Organisms for expression of a recombinant silk polypeptide include plant, algae, yeast, fungi, gram-positive, and gram-negative bacteria. In some embodiments, the host organism *is Arxula adeninivorans, Aspergillus aculeatus, Aspergillus awamori, Aspergillus ficuum, Aspergillus fumigatus, Aspergillus japonicus, Aspergillus nidulans, Aspergillus niger, Aspergillus oryzae, Aspergillus sojae, Aspergillus tubigensis, Bacillus alkalophilus, Bacillus amyloliquefaciens, Bacillus anthracis, Bacillus brevis, Bacillus circulans, Bacillus coagulans, Bacillus lautus, Bacillus lentus, Bacillus licheniformis, Bacillus methanolicus, Bacillus stearothermophilus, Bacillus subtilis, Bacillus thuringiensis, Candida boidinii, Chrysosporium lucknowense, Escherichia coli, Fusarium graminearum* , *Fusarium venenatum, Kluyveromyces lactis, Kluyveromyces marxianus, Myceliopthora thermophila*, *Neurospora crassa, Ogataea polymorpha, Penicillium camemberti, Penicillium canescens, Penicillium chrysogenum, Penicillium emersonii, Penicillium funiculosum, Penicillium griseoroseum, Penicillium purpurogenum, Penicillium roqueforti, Phanerochaete chrysosporium, Pichia angusta, Pichia methanolica, Pichia (Komagataella) pastoris, Pichia polymorpha, Pichia stipitis, Rhizomucor miehei, Rhizomucor pusillus, Rhizopus arrhizus, Streptomyces lividans, Saccharomyces cerevisiae, Schwanniomyces occidentalis, Trichoderma harzianum, Trichoderma reesei,* or *Yarrowia lipolytica.*

Additional strains that can be used as recombinant host cells are known in the art. It should be understood that the term "recombinant host cell" is intended to refer not only to the particular subject cell but to the progeny of such a cell. Because certain modifications may occur in succeeding generations due to either mutation or environmental influences, such progeny may not, in fact, be identical to the parent cell, but is still included within the scope of the term "recombinant host cell" as used herein.

### Engineered Host Cell Lines

A widely used microbe for recombinant protein production is the bacteria *Escherichia coli.* However, during culture of a strain of *E. coli,* recombinantly expressed proteins may be insoluble, resulting in poor isolation and decreased yield of recombinant proteins. Another widely used microbe is the methylotrophic yeast *Pichiapastoris. P. pastoris* grows to high cell density, provides tightly controlled methanol-inducible trans gene expression and efficiently secretes heterologous proteins in defined media. However, during culture of a strain of *P. pastoris,* recombinantly expressed proteins may be degraded before they can be collected, resulting in a mixture of proteins that includes fragments of recombinantly expressed proteins and a decreased yield of full-length recombinant proteins

In some embodiments, the modified strains with reduced protease activity described herein recombinantly express a silk-like polypeptide sequence. In some embodiments, the silk-like polypeptide sequences are 1) block copolymer polypeptide compositions generated by mixing and matching repeat domains derived from silk polypeptide sequences and/or 2) recombinant expression of block copolymer polypeptides having sufficiently large size (approximately 40 kDa) to form useful fibers by secretion from an industrially scalable microorganism. Large (approximately 40 kDa to approximately 100 kDa) block copolymer polypeptides engineered from silk repeat domain fragments, including sequences from almost all published amino acid sequences of spider silk polypeptides, can be expressed in the modified microorganisms described herein. In some embodiments, silk polypeptide sequences are matched and designed to produce highly expressed and secreted polypeptides capable of fiber formation. In some embodiments, knock-out of protease genes or reduction of protease activity in the host modified strain reduces degradation of the silk like polypeptides.

In some embodiments, to attenuate a protease activity in *Pichia pastoris,* the genes encoding these enzymes are inactivated or mutated to reduce or eliminate activity. This can be done through mutations or insertions into the gene itself of through modification of a gene regulatory element. This can be achieved through standard yeast genetics techniques. Examples of such techniques include gene replacement through double homologous recombination, in which homologous regions flanking the gene to be inactivated are cloned in a vector flanking a selectable maker gene (such as an antibiotic resistance gene or a gene complementing an auxotrophy of the yeast strain).

Alternatively, the homologous regions can be PCR-amplified and linked through overlapping PCR to the selectable marker gene. Subsequently, such DNA fragments are transformed into *Pichia pastoris* through methods known in the art, e.g., electroporation. Transformants that then grow under selective conditions are analyzed for the gene disruption event through standard techniques, e.g. PCR on genomic DNA or Southern blot. In an alternative experiment, gene inactivation can be achieved through single homologous recombination, in which case, e.g. the 5' end of the gene's ORF is cloned on a promoterless vector also containing a selectable marker gene. Upon linearization of such vector through digestion with a restriction enzyme only cutting the vector in the target-gene homologous fragment, such vector is transformed into *Pichia pastoris.* Integration at the target gene site is confirmed through PCR on genomic DNA or Southern blot. In this way, a duplication of the gene fragment cloned on the vector is achieved in the genome, resulting in two copies of the target gene locus: a first copy in which the ORF is incomplete, thus resulting in the expression (if at all) of a shortened, inactive protein, and a second copy which has no promoter to drive transcription.

Alternatively, transposon mutagenesis is used to inactivate the target gene. A library of such mutants can be screened through PCR for insertion events in the target gene.

The functional phenotype (i.e., deficiencies) of an engineered/knockout strain can be assessed using techniques known in the art. For example, a deficiency of an engineered strain in protease activity can be ascertained using any of a variety of methods known in the art, such as an assay of hydrolytic activity of chromogenic protease substrates, band shifts of substrate proteins for the selected protease, among others.

Attenuation of a protease activity described herein can be achieved through mechanisms other than a knockout mutation. For example, a desired protease can be attenuated via amino acid sequence changes by altering the nucleic acid sequence, placing the gene under the control of a less active promoter, down-regulation, expressing interfering RNA, ribozymes or antisense sequences that target the gene of interest, or through any other technique known in the art. In preferred strains, the protease activity of proteases encoded at PAS_chr4_0584 (YPS1-1) and PAS_chr3_1157 (YPS1-2) is attenuated by any of the methods described above. In some aspects, methylotrophic yeast strains, especially *Pichia pastoris* strains, wherein a *YPS1-1* and a *YPS1-2* gene have been inactivated are described. In some embodiments, additional protease encoding genes may also be knocked-out in accordance with the methods provided herein to further reduce protease activity of a desired protein product expressed by the strain.

In some embodiments, the *P. pastoris* strains disclosed herein have been modified to express a silk-like polypeptide. Methods of manufacturing preferred embodiments of silk-like polypeptides are provided in WO 2015/042164, especially at Paragraphs 114-134, incorporated herein by reference. Disclosed therein are synthetic proteinaceous copolymers based on recombinant spider silk protein fragment sequences derived from MaSp2, such as from the species *Argiope bruennichi.* Silk-like polypeptides are described that include two to twenty repeat units, in which a molecular weight of each repeat unit is greater than about 20 kDa. Within each repeat unit of the copolymer are more than about 60 amino acid residues that are organized into a number of "quasi-repeat units." In some embodiments, the repeat unit of a polypeptide described in this disclosure has at least 95% sequence identity to a MaSp2 dragline silk protein sequence.

### EXAMPLES

Below are examples of specific embodiments for carrying out the present methods described herein. The examples are offered for illustrative purposes only, and are not intended to limit the scope of the present disclosure in any way. Efforts have been made to ensure accuracy with respect to numbers used (e.g., amounts, temperatures, etc.), but some experimental error and deviation should, of course, be allowed for.

The practice of the methods described herein will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B(1992).

### Example 1: Microfluidization increases the solubility of recombinant silk protein

High physical energy was used to solubilize a model silk protein from an agglomeration of insoluble cell material in a variety of aqueous solvent conditions.

The model silk UD MiSp 64kDa is a recombinantly expressed 64 kD protein derived from the *Uloborus diversus* minor ampullate spidroin gene sequence (GenBank: DQ399332.1, SEQ ID NO: 23) concatenated to a N-terminal histidine hexamer (SEQ ID NO: 43). The protein was expressed using *Escherichia coli* C41(DE3) (Lucigen) transformed with a T7 expression vector encoding the MiSp protein. Cells were grown in a minimal medium, MiSp gene expression was induced with Isopropyl β-D-1-thiogalactopyranoside (IPTG) and lysed via homogenization. The insoluble cell lysate material was pelleted via centrifugation. A 10 to 1 (mass) ratio of 4 M Urea solution was mixed with the insoluble material for 1 hr, and the insoluble fraction was recovered by centrifugation. The homogenization equipment used produced a shear rate of about 0.7×10⁶ s⁻¹. However, this UD MiSp 64 kD model silk is known to be highly insoluble and the shear rate generated by the homogenizer was insufficient to solubilize the UD MiSp 64kDa silk.

The cell biomass and pellet containing the insoluble silk protein was resuspended in an aqueous buffer (50 mM Tris, pH 7.5) and a selected chaotrope-containing solution at a 15% w/v ratio (cell pellet mass to solution volume). The final concentration of the chaotrope was 10 M Urea, 4 M GdnHCl, 8 M GdnHCl, 3 M GdnSCN, or 6 M GdnSCN, assuming a pellet density of 1 g/l. Solutions were mixed by stirring at room temperature for at least 1 hr to disrupt gross clumps of material. 100 mL aliquots were processed with 3 passes through a F12Y interaction chamber operated at 30,000 psi (gauge pressure, M-110P, Microfluidics Inc.). A water bath was used to limit sample heating during the processing step. As a control, a separate set of 100 mL aliquots was stirred at room temperature for 3 hrs instead of processing with the microfluidizer.

The solubilization of the silk protein was assayed by centrifugation of 50 mL aliquots at 15,000 × g for 20 min at room temperature, and the supernatant and cell pellet were separated. Soluble silk protein was determined as the silk protein which remained in the supernatant after centrifugation. Insoluble silk remaining in the cell pellet was assayed by extracting the pellet with 50 mL of 5 M GdnSCN in water. The concentration of silk in the two fractions was assessed by ELISA with an anti-His6 antibody ("His6" disclosed as SEQ ID NO: 43) and the results are shown in Table 1.

**Table 1: The increase in model silk solubility after high energy processing in aqueous buffer and selected chaotrope solutions. Concentration of silk in the two fractions was determined by ELISA.**

| **Table 1** | | | | | |
|---|---|---|---|---|---|
| | | **Silk (mg/ml)** | | **Silk Yield (%)** | |
| | **Condition** | **Insoluble** | **Soluble** | **Insoluble** | **Soluble** |
| *Microfluidized 3 passes, F12Y* | | | | | |
| | 50 mM Tris | 2.5 | 0.6 | 82 | 18 |
| | 10 M Urea | 0.9 | 2.7 | 25 | 75 |
| | 4 M GdnHCl | 0.9 | 3.4 | 22 | 78 |
| | 8 M GdnHCl | 0 | 3.6 | 0 | 100 |
| | 3 M GdnSCN | 0 | 3.8 | 0 | 100 |
| | 6 M GdnSCN | 0 | 3.3 | 0 | 100 |
| | | | | | |

| *Control* | | | | | |
|---|---|---|---|---|---|
| | 50 mM Tris | 2.1 | 0 | 100 | 0 |
| | 10 M Urea | 3.7 | 0 | 100 | 0 |
| | 4 M GdnHCl | 4.4 | 0.3 | 94 | 6 |
| | 8 M GdnHCl | 2.5 | 1.8 | 58 | 42 |
| | 3 M GdnSCN | 1.4 | 2.7 | 35 | 65 |
| | 6 M GdnSCN | 0.7 | 3.9 | 16 | 84 |

For all conditions assessed, the microfluidization increased the amount of silk in the soluble fraction. Noteworthy examples are the 10 M Urea and 4 M GdnHCl conditions, where negligible silk (6% in the 4M GdnHCl or 0% in the 10M urea) was soluble in the control, but greater than 75% was soluble after the microfluidization processing. In addition, both the 3M and 6M GdnSCN buffers resulted 100% solubilized silk protein after microfluidization, whereas the control treatment with the same chaotropic concentrations did not completely solubilize the silk. Thus, the use of microfluidization lowered the chaotrope concentration required for silk protein solubilization.

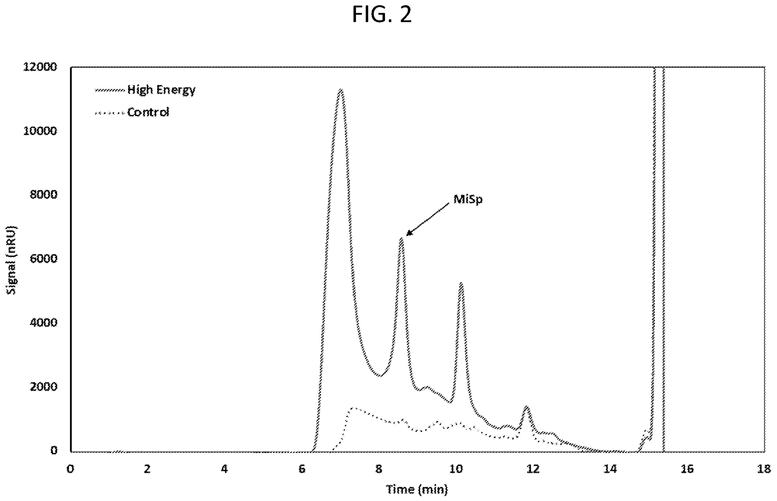

The presence of the silk protein in the soluble fraction was also confirmed by size exclusion chromatography (SEC). SEC HPLC was used to compare the soluble fraction of protein samples extracted with 10 M Urea and microfluidization (high energy line) compared to 10M urea alone (control line) **(****FIG. 2****).** The elution time of the model silk protein (MiSp peak) was identified with a purified protein standard (data not shown). The column was run with a denaturing mobile phase (5 M GdnSCN) and elutants were detected with a refractive index detector. The application of microfluidization shear force resulting in a significant increase in the solubilization and recovery of the silk protein, compared to urea alone. The purity of the solubilized silk protein was assessed by calculating the SEC peak % refractive index curve (RU) area for some samples. Purity for the selected samples is shown in **Table 2.**

| **Table 2** | | | |
|---|---|---|---|
| | | **Purity (% Area SEC)** | |
| | **Condition** | **Insoluble** | **Soluble** |
| *Microfluidized 3 passes, F12Y* | | | |
| | 50 mM Tris | 40 | |
| | 10 M Urea | 55 | 18 |
| | 4 M GdnHCl | 56 | 23 |
| | 8 M GdnHCl | | 25 |
| | 3 M GdnSCN | | |
| | 6 M GdnSCN | | 25 |
| | | | |

| *Control* | | | |
|---|---|---|---|
| | 50 mM Tris | 35 | |
| | 10 M Urea | 43 | 9 |
| | 4 M GdnHCl | 41 | 13 |
| | 8 M GdnHCl | | 30 |
| | 3 M GdnSCN | | |
| | 6 M GdnSCN | | 36 |

### Example 2: Shear rate pressure optimization

Next, the interaction chamber size and pressure of the microfluidization was altered to assess silk solubilization.

Insoluble cell biomass containing the silk protein was prepared as described **Example 1** and suspended in urea to a final concentration of 10 M urea. Samples were processed at two different shear rates, controlled by the interaction chamber type and operating pressure as described by the manufacturer (Microfluidics Processor User Guide, Microfluidics, Inc). Specifically, predicted shear rates of 6.5 × 10⁶ s⁻¹ (G10Z interaction chamber, 23,000 psi, Microfluidics Inc. LM10) or 9.5 × 10⁶ s⁻¹ (F12Y interaction chamber, 30,000 psi, Microfluidics Inc. M-110P) were targeted. The solubilization of the silk protein was assayed by the centrifugation protocol described in **Example 1.** The concentration of silk in the soluble or insoluble fractions was assessed by SEC HPLC as previously described: the silk protein was measured by the area of the SEC refractive index peak, using Bovine Serum Albumin protein standards for calculating silk concentrations.

**Table 3** shows the solubilization of the silk after the two treatments. The increase in shear rates to 9.5 × 10⁶ s⁻¹ increased the yield of silk protein solubilized by 40% compared to the sample processed at the lower rate of 6.5 × 10⁶ s⁻¹ (47% yield using 23,000 psi compared to 66% yield using 30,000 psi). Thus, a higher shear rate resulted in increased silk protein solubilization and recovery.

**Table 3: The effect of varying the shear rate when solubilizing the silk protein with 10 M Urea. Silk concentration was estimated from SEC HPLC peak area. Yield is the amount of silk protein recovered as normalized to the total amount of silk protein in the starting material. The total amount of silk protein in the starting material was extracted via incubation with 5 M GdnSCN.**

| **Table 3** | | | | | |
|---|---|---|---|---|---|
| | | **Silk Yield (%)** | | **Silk (mg/ml)** | |
| | **Condition** | **Insoluble** | **Soluble** | **Insoluble** | **Soluble** |
| *G10Z 23,000 psi* | | | | | |
| | 10 M Urea | 53 | 47 | 2.0 | 1.8 |
| | | | | | |
| *F12Y 30,000 psi* | | | | | |
| | 10 M Urea | 34 | 66 | 1.2 | 2.3 |

While the invention has been particularly shown and described with reference to a preferred embodiment and various alternate embodiments, it will be understood by persons skilled in the relevant art that various changes in form and details can be made therein without departing from the spirit and scope of the invention.

All references, issued patents and patent applications cited within the body of the instant specification are hereby incorporated by reference in their entirety, for all purposes.

### EMBODIMENTS

The invention is further described with reference to the following numbered embodiments:
**1.** A method of isolating a recombinant spider silk protein from a host cell, comprising:
   a. providing an insoluble mass comprising a recombinant spider silk protein;
   b. adding the insoluble mass to an aqueous solution comprising a solvent;
   c. applying a shear force to the aqueous solution comprising the insoluble mass thereby solubilizing the recombinant spider silk protein in the aqueous solution.
**2.** The method of embodiment 1, wherein the shear force is applied via microfluidization.
**3.** The method of embodiment 2, wherein the microfluidization generates shear rates of about 6×10⁶ s⁻¹ to 10×10⁶ s⁻¹.
**4.** The method of embodiment 2, wherein the microfluidization generates shear rates of at least about 6×10⁶ s⁻¹.
**5.** The method of embodiment 2, wherein the microfluidization generates shear rates of at least about 10×10⁶ s⁻¹.
**6.** The method of embodiments 2-5, wherein the microfluidization is performed at between 20,000 psi to 30,000 psi.
**7.** The method of embodiment 6, wherein the microfluidization is performed at 30,000 psi.
**8.** The method of embodiment 6, wherein the microfluidization is performed at 23,000 psi.
**9.** The method of any of the above embodiments, wherein the microfluidizer an M-110P or an LM10 microfluidizer.
**10.** The method of embodiment 9, wherein the microfluidizer comprises a G10Z interaction chamber.
**11.** The method of embodiment 9, wherein the microfluidizer comprises an F12Y interaction chamber.
**12.** The method of any of the above embodiments, wherein the shear force is applied at least twice.
**13.** The method of embodiment 12, wherein the shear force is applied at least three times.
**14.** The method of embodiment 12, wherein the shear force is the same in the at least two applications.
**15.** The method of embodiment 12, wherein the shear force is different in the at least two applications.
**16.** The method of any of the above embodiments, wherein the insoluble mass is derived from a cell culture comprising a host cell, wherein the host cell expresses the recombinant spider silk protein.
**17.** The method of embodiment 16, further comprising collecting the insoluble mass derived from the cell culture, wherein the insoluble mass comprises the recombinant spider silk protein.
**18.** The method of any one of embodiments 1-17, wherein the solvent is a chaotropic agent.
**19.** The method of embodiment 18, wherein the chaotropic agent is urea, guanidine thiocyanate (GdnSCN), or guanidine chloride (GdnHCL).
**20.** The method of any one of embodiments 1-19, wherein the insoluble mass is added to the aqueous solution at about a 5%, 10%, 15%, 20%, 25%, or 30% insoluble mass to solvent volume.
**21.** The method of embodiment 19 or 20, wherein the chaotropic agent is present in the aqueous solution at a concentration from 0.1 - 10M.
**22.** The method of embodiment 19 or 20, wherein the aqueous solution comprises about 10M urea, about 4M - 8M GdnHCl, or about 3M - 6M GdnSCN.
**23.** The method of embodiment 19 or 20, wherein the aqueous solution comprises a chaotropic activity of no more than an aqueous solution comprising 10M urea, an aqueous solution comprising 8M GdnHCl, or an aqueous solution comprising 6M GdnSCN.
**24.** The method of embodiment 23, wherein the chaotropic activity is quantified using an agar-gelation assay.
**25.** The method of embodiment 20, wherein the aqueous solution comprises about a 15% insoluble portion mass to an 85% volume of 3M GdnSCN.
**26.** The method of embodiment 20, wherein the aqueous solution comprises about a 15% insoluble portion mass to an 85% volume of 4M GdnHCl.
**27.** The method of embodiment 20, wherein the aqueous solution comprises about a 15% insoluble portion mass to an 85% volume of 10M urea.
**28.** The method of any one of embodiments 1-27, wherein the insoluble mass is incubated between 20°C and 30°C.
**29.** The method of embodiment 28, wherein the insoluble mass is incubated at room temperature.
**30.** The method of embodiment 28, wherein the insoluble mass is incubated at no more than 30°C.
**31.** The method of any one of embodiments 1-30, wherein the insoluble portion is incubated in the aqueous solution comprising the solvent for 60 to 120 minutes.
**32.** The method of any one of embodiments 1-31, wherein the insoluble mass comprises a cell pellet.
**33.** The method of any one of embodiments 1-32, wherein collecting the insoluble mass derived from the cell pellet comprises lysing the host cell.
**34.** The method of embodiment 33, wherein lysing comprises heat treatment, chemical treatment, shear disruption, physical homogenization, sonication, or chemical homogenization.
**35.** The method of embodiments 33 to 34, wherein collecting the insoluble mass of the cell culture further comprises centrifuging the lysed cell to obtain a first cell pellet.
**36.** The method of any one of embodiments 1-35, wherein collecting the insoluble mass further comprises:
   a. incubating the cell pellet with a solution comprising 4M urea at a 10: 1 urea volume to pellet mass ratio; and
   b. centrifuging the solution comprising 4M urea to obtain a second cell pellet prior to incubating the second cell pellet in the aqueous solution comprising a solvent.
**37.** The method of any one of embodiments 1-36, further comprising isolating the recombinant spider silk protein from the aqueous solution, thereby producing an isolated recombinant spider silk protein.
**38.** The method of any one of embodiments 1-37, wherein the recombinant spider silk protein is a highly crystalline silk protein, a high beta sheet content silk protein, or a low solubility silk protein.
**39.** The method of embodiment 38, wherein the recombinant spider silk protein comprises the *Uloborus diversus* MiSP protein as set forth in SEQ ID NO: 23.
**40.** The method of embodiment 38, wherein the recombinant spider silk protein has a solubility threshold of less than 90%, 80%, 70%, 60%, or 50% in a non-chaotropic solvent.
**41.** The method of any one of embodiments 1-40, wherein the cell culture comprises a fungal, a bacterial, or a yeast cell.
**42.** The method of any one of embodiments 1-41, wherein the bacterial cell is *Escherichia coli.*
**43.** The method of any of the above embodiments, wherein an amount of isolated recombinant spider silk protein is measured using an ELISA.
**44.** The method of any one of embodiments 1-43, wherein an amount of isolated recombinant spider silk protein is measured using Size Exclusion Chromatography.
**45.** The method of any one of embodiments 1-44, wherein the isolated recombinant spider silk protein is full-length recombinant spider silk protein.
**46.** The method of embodiment 45, wherein the isolated recombinant spider silk protein comprises at least 60%, at least 65%, at least 70%, at least 75%, at least 80%, at least 85%, at least 90%, or at least 95% full-length recombinant spider silk protein.
**47.** The method of embodiment 45, wherein an amount of full-length recombinant spider silk protein is measured using an ELISA.
**48.** The method of embodiment 45, wherein an amount of full-length recombinant spider silk protein is measured using Size Exclusion Chromatography.
**49.** The method of any one of embodiments 1-48, wherein the purity of the isolated recombinant spider silk protein is 5-10%, 10-15%, 15-20%, 20-25%, 25-30%, 30-35%, 35-40%, 45-50%, 50-55%, 55-60%, 60-65%, 65-70%, 70-75%, 75-80%, 80-85%, 85-90%, 09-95%, or 95-100%.
**50.** A method of isolating a recombinant spider silk protein from a host cell, comprising:
   a. providing an insoluble mass comprising a recombinant spider silk protein;
   b. adding the insoluble mass to an aqueous solution comprising a solvent, wherein the aqueous solution comprises 15% (w/v) insoluble portion in a final 10M urea concentration;
   c. applying a shear force via microfluidization to the aqueous solution comprising the insoluble mass thereby solubilizing the recombinant spider silk protein in the aqueous solution; and
   d. isolating the recombinant spider silk protein from the aqueous solution, thereby producing an isolated recombinant spider silk protein.
**51.** A method of isolating a recombinant spider silk protein from a host cell, comprising:
   a. providing an insoluble mass comprising a recombinant spider silk protein;
   b. adding the insoluble mass to an aqueous solution, wherein the aqueous solution comprises about a 15% (w/v) insoluble portion in a final 10M urea concentration;
   c. applying a shear force via microfluidization, wherein the shear force is about 10×10⁶ s⁻¹, to the aqueous solution thereby solubilizing the recombinant spider silk protein in the aqueous solution; and
   d. isolating the recombinant spider silk protein from the aqueous solution, thereby producing an isolated recombinant spider silk protein.
**52.** A composition comprising a recombinant spider silk protein produced by the method of any one of one of embodiments 1-51.
**53.** The composition of embodiment 52, wherein the recombinant spider silk comprises at least 55%, at least 60%, at least 65%, at least 70%, at least 75%, at least 80%, at least 85%, at least 90%, at least 95%, or 100% full length recombinant spider silk.
**54.** A silk fiber comprising a recombinant spider silk protein produced by the method of any one of embodiments 1-53.

## Claims

1. A method of isolating a recombinant spider silk protein from a host cell, comprising:
a. providing an insoluble mass comprising a recombinant spider silk protein;
b. adding the insoluble mass to an aqueous solution comprising a solvent;
c. applying a shear force to the aqueous solution comprising the insoluble mass thereby solubilizing the recombinant spider silk protein in the aqueous solution.

2. The method of claim 1, wherein the shear force is applied via microfluidization, optionally wherein (i) the microfluidization generates shear rates of at least about 6×10⁶ s⁻¹ or at least about 10×10⁶ s⁻¹, e.g., about 6×10⁶ s⁻¹ to 10×10⁶ s⁻¹; and/or (ii) the microfluidization is performed at between 20,000 psi to 30,000 psi, e.g., at 23,000 psi or at 30,000 psi.

3. The method of claim 1 or 2, wherein the microfluidizer is an M-110P or an LM10 microfluidizer, optionally wherein the microfluidizer comprises a G10Z interaction chamber or an F 12Y interaction chamber.

4. The method of any one of the preceding claims, wherein the shear force is applied at least twice, optionally wherein: (i) the shear force is applied at least three times, (ii) the shear force is the same in the at least two applications, or (iii) the shear force is different in the at least two applications.

5. The method of any one of the preceding claims, wherein the insoluble mass is derived from a cell culture comprising a host cell, wherein the host cell expresses the recombinant spider silk protein, optionally wherein the method further comprises collecting the insoluble mass derived from the cell culture, wherein the insoluble mass comprises the recombinant spider silk protein, optionally wherein the cell culture comprises a fungal, a bacterial, or a yeast cell, optionally wherein the bacterial cell is *Escherichia coli.*

6. The method of any one of the preceding claims, wherein the solvent is a chaotropic agent, optionally wherein the chaotropic agent is urea, guanidine thiocyanate (GdnSCN), or guanidine chloride (GdnHCL).

7. The method of any one of the preceding claims, wherein the insoluble mass is added to the aqueous solution at about a 5%, 10%, 15%, 20%, 25%, or 30% insoluble mass to solvent volume, optionally wherein the aqueous solution comprises about a 15% insoluble mass to an 85% volume of 3M GdnSCN, about a 15% insoluble mass to an 85% volume of 4M GdnHCl, or about a 15% insoluble mass to an 85% volume of 10M urea.

8. The method of claim 6 or 7, wherein the chaotropic agent is present in the aqueous solution at a concentration from 0.1 - 10M, optionally wherein the aqueous solution comprises about 10M urea, about 4M - 8M GdnHCl, or about 3M - 6M GdnSCN, or the aqueous solution comprises a chaotropic activity of no more than an aqueous solution comprising 10M urea, an aqueous solution comprising 8M GdnHCl, or an aqueous solution comprising 6M GdnSCN, optionally wherein the chaotropic activity is quantified using an agar-gelation assay.

9. The method of any one of the preceding claims, wherein the insoluble mass is incubated (i) between 20°C and 30°C, optionally at room temperature, and/or (ii) in an aqueous solution comprising the solvent for 60 to 120 min.

10. The method of any one of the preceding claims, wherein:
(i) the insoluble mass comprises a cell pellet, optionally wherein collecting the insoluble mass derived from the cell pellet comprises lysing the host cell, optionally wherein:
a. lysing comprises heat treatment, chemical treatment, shear disruption, physical homogenization, sonication, or chemical homogenization; and/or
b. collecting the insoluble mass of the cell culture further comprises centrifuging the lysed cell to obtain a first cell pellet, and/or
(ii) collecting the insoluble mass further comprises:
a. incubating the cell pellet with a solution comprising 4M urea at a 10: 1 urea volume to pellet mass ratio; and
b. centrifuging the solution comprising 4M urea to obtain a second cell pellet prior to incubating the second cell pellet in the aqueous solution comprising a solvent.

11. The method of any one of the preceding claims, further comprising isolating the recombinant spider silk protein from the aqueous solution, thereby producing an isolated recombinant spider silk protein.

12. The method of any one of the preceding claims, wherein the recombinant spider silk protein is a highly crystalline silk protein, a high beta sheet content silk protein, or a low solubility silk protein, optionally wherein the recombinant spider silk protein comprises the *Uloborus diversus* MiSP protein as set forth in SEQ ID NO: 23 or has a solubility threshold of less than 90%, 80%, 70%, 60%, or 50% in a non-chaotropic solvent.

13. The method of any one of the preceding claims, wherein the isolated recombinant spider silk protein is full-length recombinant spider silk protein, optionally wherein the isolated recombinant spider silk protein comprises at least 60%, at least 65%, at least 70%, at least 75%, at least 80%, at least 85%, at least 90%, or at least 95% full-length recombinant spider silk protein.

14. The method of any one of the preceding claims, wherein the purity of the isolated recombinant spider silk protein is 5-10%, 10-15%, 15-20%, 20-25%, 25-30%, 30-35%, 35-40%, 45-50%, 50-55%, 55-60%, 60-65%, 65-70%, 70-75%, 75-80%, 80-85%, 85-90%, 09-95%, or 95-100%.

15. A composition or a silk fiber comprising a recombinant spider silk protein produced by the method of any one of the preceding claims.
